(19) Europäisches Patentamt
European Patent Office
Office européen des brevets

(11) **EP 3 404 567 A1**

(12) **EUROPEAN PATENT APPLICATION**

(43) Date of publication:
**21.11.2018 Bulletin 2018/47**

(51) Int Cl.:
**G06F 19/10** *(2011.01)*          **G06F 19/24** *(2011.01)*
**G06F 19/28** *(2011.01)*

(21) Application number: **17171925.5**

(22) Date of filing: **19.05.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fujitsu Limited**
**Kawasaki-shi, Kanagawa 211-8588 (JP)**

(72) Inventors:
• **NOVÁCEK, Vit**
**Co. Galway (IE)**

• **COSTABELLO, Luca**
**Co. Galway (IE)**
• **VANDENBUSSCHE, Pierre-Yves**
**Co. Galway (IE)**
• **MITSUISHI, Yutaka**
**Co. Galway (IE)**

(74) Representative: **Haseltine Lake LLP**
**Lincoln House, 5th Floor**
**300 High Holborn**
**London WC1V 7JH (GB)**

(54) **A SYSTEM AND A METHOD FOR DISCOVERY OF PREDICTED SITE-SPECIFIC PROTEIN PHOSPHORYLATION CANDIDATES**

(57)     A method for discovery of predicted site-specific protein phosphorylation statements, the statements linking a kinase, a predicted phosphorylation site, and a protein substrate, the method comprising: using data from a phosphorylation data set storing known site-specific statements each in a site-specific format linking a kinase, a phosphorylation site and a protein substrate; a kinase family database; and a protein sequence database to produce motif-based phosphorylation statements each in a generalised format linking a kinase, a motif in the form of a generalised scoring matrix for amino acids at a phosphorylation site and to either side of the phosphorylation site; and a protein substrate; generating negative statements from the motif-based statements; generating candidate statements in the generalised format by combining the motif-based statements; generating negative candidate statements by combination of the candidate statements with the negative statements; using the candidate statements and negative candidate statements in a statistical relational learning model to score the candidate statements and retain predicted motif-based phosphorylation statements in the generalised format; receiving data inputs from the protein sequence database and the predicted motif-based phosphorylation statements in the generalised format and producing predicted site-specific phosphorylation statements in the site-specific format.

Figure 1B

**Description**

**Technological Field**

The present invention relates to a system and a method for the discovery of site-specific phosphorylation reactions between proteins. Embodiments are based on converting phosphorylation data into knowledge graphs, augmenting the graphs by relevant contextual information and consequent predictions using latent feature models.

The invention has multiple applications in the area of system and molecular biology and pharmacology. It is primarily used for human proteins and kinases, but may be applied to other species if the appropriate data is available.

**Background**

Phosphorylation (the addition of phosphoryl group ($PO_3$) to a molecule) is a ubiquitous protein interaction driving many important signalling pathways in living cells. Phosphorylation often regulates for instance cell growth, division or programmed death. Disruptions of these regulatory processes are the main culprit of many serious diseases such as cancer. Therefore phosphorylation is one of the key topics studied in system and molecular biology, as well as in pharmacology.

Yet the rate of discoveries of new phosphorylation reactions and their function is still rather slow. One of the main reasons is the large number of combinations of possible site-specific phosphorylations; exploring this space is hard even with modern high-throughput technologies. Another reason is that there are experimental difficulties in establishing which of the candidate reactions actually do happen in cells and lead to a significant effect.

Automated prediction of site-specific phosphorylation candidates is much desired as it may substantially reduce the space of possible phosphorylations to be experimentally verified. This may lead to much increased rate of discoveries in cellular signalling, which may in turn contribute to more efficient treatment of diseases like cancer.

According to an embodiment of a first aspect of the invention, there is provided a system (or apparatus) for discovery of predicted site-specific protein phosphorylation statements, the statements linking a predicted phosphorylation site, a protein substrate and a kinase, the system comprising:

a motif converter which is to receive inputs from:

a phosphorylation data set storing known site-specific statements each in a site-specific format linking a kinase, a phosphorylation site and a protein substrate;
a kinase family database; and
a protein sequence database,

the motif converter being arranged to produce motif-based phosphorylation statements each in a generalised format linking a kinase, a motif in the form of a generalised scoring matrix for amino acids at a phosphorylation site and to either side of the phosphorylation site; and a protein substrate;
a link predictor to predict new motif-based statements, the link predictor including:

a negative motif-based statements generator to generate negative statements from the motif-based statements;
a candidate generator to generate candidate statements in the generalised format by combination of the motif-based statements and to generate negative candidate statements by combination of the candidate statements with the negative statements;
a relational learner to use the candidate statements and the negative candidate statements in a statistical relational learning model to score the candidate statements and retain predicted motif-based phosphorylation statements in the generalised format; and
a site-specific converter to receive inputs from the protein sequence database and to receive the predicted motif-based phosphorylation statements in the generalised format and which is arranged to produce predicted site-specific phosphorylation statements in the site-specific format.

The system obtains predicted site-specific protein phosphorylation statements, the statements linking a kinase, a predicted phosphorylation site, and a protein substrate. The site-specific statements with these three entities may be represented as <K (kinase), L or site, S (substrate)>. The link represents the predicted phosphorylation interaction at the given site on the substrate using the kinase.

The motif converter takes a phosphorylation data set storing known site-specific statements each in a site-specific format linking a kinase, a phosphorylation site and a protein substrate and uses additional information from a kinase family database and a protein sequence database to convert the site-specific statements to motif-based phosphorylation statements. These are in a "generalised" format in the sense that they link a kinase, a motif in the form of a generalised scoring matrix for amino acids at a phosphorylation site and to either side of the phosphorylation site; and a protein substrate. The generalised statements with these three entities may be represented as <K (kinase), M (motif), S (substrate)>. In embodiments, the motif is provided before the generalised statements are created and may be viewed as a consensus sequence pattern which is derived by grouping of the input site specific statements. The motif is learned from a group of phosphorylation sites and their amino acid contexts (in general, protein sequence motifs are learned from a set of protein sequences, of which the sites and their contexts are a sub-type). Hence the motif creation may be seen more as a group process than as a site-by-site process.

To predict new motif-based statements, the link predictor includes a negative motif-based statements generator to generate negative statements from the motif-based statements, and a candidate generator, which creates candidate statements in the generalised format by combination of the motif-based statements. The candidate generator also generates negative candidate statements (indicating that phosphorylation is not expected to occur at the stated motif on the stated substrate using the stated kinase. The negative candidates are generated by combination of the motif-based candidate statements and the negative motif-based statements).

The link predictor also includes a relational learner to use the candidate statements and the negative candidate statements in a statistical relational learning model to score and maybe also rank the candidate statements (and potentially the negative candidate statements). This allows retention of higher scored/ranked statements as predicted statements.

Finally, the site-specific converter uses the protein sequence database to convert the predicted motif-based phosphorylation statements into the site-specific format. This is the format required by users of the system, because it allows identification of predicted phosphorylation locations along with the substrate and kinase, which may then be further explored.

The motif converter may

associate each site-specific statement with a kinase family in the kinase family database;

use the protein sequence database to form a context sequence of an amino acid at the phosphorylation site for each site-specific statement and a plurality of amino acids to either side of the phosphorylation site;

group the context sequences by kinase family;

for the context sequences in each kinase family, compute one or more position-specific scoring matrices each of which provides a score for each amino acid at each position of the context sequence length, each position-specific scoring matrix defining a motif;

for each site-specific statement, compute the conformance between the context sequence for that site-specific statement and each motif in the kinase family in which the context sequence is grouped;

for each site-specific statement, create a motif-based statement linking the kinase, the best performing motif and the protein substrate.

The context sequence and motif are of the same length, which may be from 3 to 21 amino acids to either side of the amino acid (and the amino acid at the phosphorylation site), and may be from 7 to 15 amino acids to either side. In fact, any integer number above zero may work with the technique, so potentially just the location amino acid and 1 acid either side could be used. However the results vary quite widely: this length is an important hyperparameter of the methods, and it appears that a length 7 or 15 amino acids to either side may be the most suitable in most circumstances. Very short context windows are not likely to bring good results. The window need not be centred on the phosphorylation site. Normally the number of amino acids considered to the left and to the right of the phosphorylation site is equal, but there may be more amino acids to one side, for example there may be 3 on the left and 5 on the right to produce a context sequence of full length 9.

In more detail, the negative statements generator is to create negative statements by one or more of: replacing the substrate in a motif-based statement (<K, M, S>) with a different substrate and replacing the kinase in a motif-based statement with a kinase from a different kinase family. Thus there are two methods of making negative statements.

In the first method, the substrate S in a motif-based statement <K, M, S> is replaced by a different substrate S' in that for each phosphorylation site on the different substrate, the context sequence is compared with the motif of the motif-based statement, and if the conformance is negative, the negative statement <K, M, S'> is provided. As an aside, it is noted that the order of the entities in the statements used herein is arbitrary and that any order is acceptable and equivalent if used consistently.

In the second method, the kinase K in a motif-based statement <K, M, S> is replaced by a different kinase K' in that for each kinase which is not in the same family as kinase K, a statement <K', M, S> is provided.

In the generation of negative statements, the negative statements and the motif-based statements may be compared and any statement that appears both as one of the negative statements and as one of the motif-based statements is deleted from the negative statements.

A negative statements filter in the candidate generator may then, for each kinase and motif in a candidate statement, create negative candidate statements as the combination of the kinase, the motif and all substrates associated with the kinase and the motif in the negative statements.

The negative statements filter may also, for each motif and substrate in a candidate statement, create negative candidate statements as the combination of the motif, the substrate and all kinases associated with the substrate and the motif in the negative statements. Hence there are two methods of creating negative candidate statements which may be used,
The negative statements filter may discard from the negative candidate statements any statement that appears both as one of the negative candidate statements and as one of the candidate statements.

The relational learner may learn a model from the motif-based statements and negative statements and may then use the model to score the candidate statements and the negative candidate statements.

The candidate generator may create candidate statements as the combination of every kinase included in the motif-based statements with every motif included in the motif-based statements and every substrate included in the motif-based statements (a Cartesian product) minus the motif-based statements themselves. The candidates are not used in the training of the model, but only scored later, once the model has been created.

To do this, the relational learner may score and rank a candidate statement and all the negative candidate statements with the same kinase and motif or with the same kinase and substrate and output the rank and score of the candidate statement.

A protein statements generator may receive input from a protein context database linking proteins by their interactions and may produce protein context statements, for use in the link predictor. This additional information enriches the knowledge used in the system.

The site-specific converter may:

input the predicted motif-based phosphorylation statements;
for each predicted motif-based phosphorylation statements extract a list of possible phosphorylation sites from a phosphorylation sites database;
for each phosphorylation site, extract the site context sequence from the protein sequence database and compute the conformance of the site context sequence with the motif in the predicted motif-based phosphorylation statement;
if the conformance for the phosphorylation site is above a predefined score (for example zero), produce a predicted site-specific phosphorylation statement in the site-specific format linking the kinase and the substrate in the predicted motif-based statement with the phosphorylation site.

According to an embodiment of a second aspect of the invention, there is provided a computer-implemented method for discovery of predicted site-specific protein phosphorylation statements, the statements linking a predicted phosphorylation site, a protein substrate and a kinase, the method comprising:

using data from:

a phosphorylation data set storing known site-specific statements each in a site-specific format linking a kinase, a phosphorylation site and a protein substrate;
a kinase family database; and
a protein sequence database,

to produce motif-based phosphorylation statements each in a generalised format linking a kinase, a motif in the form of a generalised scoring matrix for amino acids at a phosphorylation site and to either side of the phosphorylation site; and a protein substrate;
generating negative statements from the motif-based statements;
generating candidate statements in the generalised format by combining the motif-based statements;
generating negative candidate statements by combination of the candidate statements with the negative statements;
using the candidate statements and negative candidate statements in a statistical relational learning model to score the candidate statements and retain predicted motif-based phosphorylation statements in the generalised format;
receiving data inputs from the protein sequence database and the predicted motif-based phosphorylation statements in the generalised format and producing predicted site-specific phosphorylation statements in the site-specific format.

A method according to preferred embodiments of the present invention may comprise any combination of the apparatus or computer program aspects. Methods or computer programs according to further embodiments may be described as computer-implemented in that they require processing and memory capability.

The apparatus according to preferred embodiments is described as configured or arranged to, or simply "to" carry out certain functions. This configuration or arrangement could be by use of hardware or middleware or any other suitable system. In preferred embodiments, the configuration or arrangement is by software.

Thus according to one aspect there is provided a program which, when loaded onto at least one computer configures the computer to become the apparatus according to any of the preceding apparatus definitions or any combination thereof.

According to a further aspect there is provided a program which when executed on at least one computer configures the at least one computer to carry out the method steps according to any of the preceding method definitions or any combination thereof.

In general the computer may comprise the elements listed as being configured or arranged to provide the functions defined. For example this computer may include memory, processing, and a network interface.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The invention is described in terms of particular embodiments. Other embodiments are within the scope of the following claims. For example, the steps of the invention may be performed in a different order and still achieve desirable results. Multiple test script versions may be edited and invoked as a unit without using object-oriented programming technology; for example, the elements of a script object may be organized in a structured database or a file system, and the operations described as being performed by the script object may be performed by a test control program.

Elements of the invention have been described using the terms "converter", "predictor", "generator", "learner" etc. The skilled person will appreciate that such functional terms and their equivalents expressed in terms of units may refer to parts of the system that are spatially separate but combine to serve the function defined. Equally, the same physical parts of the system may provide two or more of the functions defined.

For example, separately defined means may be implemented using the same memory and/or processor as appropriate.

Preferred features of the present invention will now be described, purely by way of example, with references to the accompanying drawings, in which:-

Figure 1A is a block diagram of a system overview;
Figure 1B is a flow chart of a method in a general embodiment;
Figure 1C is a block diagram of a more detailed system overview;
Figure 2 is a block diagram including main system components in a link prediction unit/predictor of a general embodiment of the invention;
Figure 3 is a diagram of a converter to motif-based statements;
Figure 4 is a flow chart of a motif discovery unit;
Figure 5 is flow chart of a motif-based statement generator;
Figure 6 is block diagram of a knowledge base enrichment unit to add protein context statements;
Figure 7 is a flow chart of protein context statements of a generation unit;
Figure 8 is a block diagram of a link prediction unit;
Figure 9 is a flow chart of a negative motif-based statements generation unit;
Figure 10 is a flow chart of a negative protein context statements generation unit;
Figure 11 is a block diagram of a candidate generation unit;
Figure 12 is a flow chart of a positive candidate statements generation unit;
Figure 13 is a flow chart of a negative candidate statements generation unit;
Figure 14 is a flow chart of a relational learning unit;
Figure 15 is a block diagram of a conversion to site-specific statements unit;

Figure 16 is a flow chart conversion to site-specific statements unit; and

Figure 17 is a block diagram of a computing device to carry out methods of embodiments.

Most current approaches to phosphorylation prediction tend to have a conceptually similar approach to the phosphorylation prediction problem. They typically start from a data set on potential phosphorylation sites (which are relatively easy and cheap to obtain in large quantities due to techniques like mass spectrometry). Then they identify substrates that contain potential phosphorylation sites (usually focusing only on relatively few possible site/substrate combinations). The last step is identification of kinases (enzymes used to phosphorylate proteins) that could target these sites in the substrates. The specific prior arts differ in the exact methodology of the particular steps, but not so much in the general approach. The main reason for use of the same general approach is that until relatively recently, this has been possibly the only plausible approach to the problem as there were no other types of data applicable to predicting phosphorylations. However, in the last decade, several large, machine-readable and manually curated phosphorylation networks datasets have become available. These data sets present a summary of current knowledge of all site-specific phosphorylation relations in the form of a knowledge graph, also known as a graph database. This is a valuable source of information since the latent structure of such knowledge bases may typically yield many new discoveries as has been shown in other domains, such as in knowledge base completion or knowledge fusion. The inventors have realised that this knowledge graph may also be easily combined with other relevant sources of contextual information (such as protein interaction networks) which is an additional advantage over the data sets typically used by prior art for phosphorylation prediction. Yet there is no existing technique capable of tapping into this type of information to predict new phosphorylations at the moment.

Graph databases/knowledge graphs may be used to maintain large "semantic networks" that may store large amounts of structured and unstructured data in various fields. There are several types of graph representations. Graph data may be stored in memory as multidimensional arrays, or as symbols linked to other symbols. Another form of graph representation is the use of "tuples," which are finite sequences or ordered lists of objects, each of a specified type, such as a Uniform Resource Identifier, URI. The knowledge base itself may well use tuples for storage, as do many data stores. A tuple containing n objects is known as an "n-tuple," where n may be any non-negative integer greater than zero. A tuple of length 2 (a 2-tuple) is commonly called a pair, a 3-tuple is called a triple, a 4-tuple is called a quadruple, and so on.

Triples are one of the most commonly used tuples. Each triple may be visualised as a subject-predicate-object (s-p-o) statement or relation. The triples may be Resource Description Framework (RDF) triples. The Resource Description Framework is a general method for conceptual description or modelling of information that is a standard for semantic networks. Standardising the modelling of information in a semantic network allows for interoperability between applications operating on a common semantic network. RDF maintains a vocabulary with unambiguous formal semantics, by providing the RDF Schema (RDFS) as a language for describing vocabularies in RDF.

An RDF graph/triples may be queried (accessed) using the SPARQL Protocol and RDF Query Language (SPARQL). It was standardized by the RDF Data Access Working Group (DAWG) of the World Wide Web Consortium, and is considered a key semantic web technology.

The inventors have come to the realisation that the prediction of new phosphorylations from knowledge graphs that summarise the relevant biological knowledge may be associated with the following technical problems (which the inventors have identified):

**Technical Problem A) Neglecting the potential of known phosphorylations and related contextual information represented as knowledge graphs.** Phosphorylation knowledge bases and protein networks present graph statistical patterns and regularities such as autocorrelation, block structure, global and long-range statistical dependencies over chains of proteins made of different relation types. These patterns may be used as foundations for effective phosphorylation predictions from known, curated data sets. However, designing predictive models that learn as many combinations of such patterns as possible is not straightforward in the phosphorylation context. This is due to the fact that for successful solutions, approaches from diverse fields of biology, bioinformatics and relational machine learning have to be combined in a non-obvious way.

**Technical Problem B) Lack of tools that can learn from context-dependent networks.** Phosphorylation knowledge graphs are made of untyped binary relations that are, however, dependent on contexts (i.e., the phosphorylation sites). This prevents straightforward processing with state-of-the-art machine learning models, since these models are designed to work on typed binary relations without contexts. Therefore, the context information has to be converted into relation types in order for the models to work. In this context, typed relations are pairwise relations between entities A and B (e.g., proteins) where the relationship is of a specific type R (e.g., phosphorylation or inhibition), and untyped relations are pairwise relations between entities A and B (e.g., proteins) where the type of relationship is not specified (i.e., the statement says nothing beyond the fact that A and B are somehow associated)

**Technical Problem C) Inappropriateness of standard negative generation techniques for phosphorylation knowledge graphs.** Machine learning models typically used for mining knowledge graphs (such as statistical relational learning) require negative examples. These are difficult to establish in many realistic use cases due to the Open World Assumption (missing knowledge should not be treated as negative, but merely as unknown). This is also the case of phosphorylation (one cannot simply take statements not represented in the current data sets as negatives as they may possibly represent phosphorylations that simplify have not been discovered yet).

To the best of the inventors' knowledge, there is no bioinformatics technique capable of tapping into phosphorylation knowledge graphs to predict new phosphorylations. Additionally, no one has solved the technical problems presented above. More specifically:

Technical Problem A is not solved because the prior art adopts the traditional approach to phosphorylation prediction (starting with the sites and associating likely substrates and kinases with them). The graph-like structures representing phosphorylations are often an output of the prior art methods, but not a primary input. Therefore these works cannot take into account the available manually curated knowledge represented as phosphorylation networks. They also cannot easily combine this data with other graph-like data (such as protein interactions) that may be very beneficial for making the predictions.

No other work tackles phosphorylation prediction with a graph-based approach. Therefore there are also no existing solutions to transform the untyped, context-dependent phosphorylation data sets into actionable knowledge graphs composed of typed, context-independent relationships. Thus there is no current proposed prior art solution for Technical Problem B.

Techniques for generating plausible negative examples under Open World Assumption have been proposed in the state-of-the-art. However, these techniques are generally not reliable in the phosphorylation context, since they may theoretically produce some negative statements that are positive in real life, which may seriously hamper the performance of the prediction models. While there are existing approaches for generating negative examples of phosphorylation sites, there is no prior art concerning negative examples of the whole phosphorylation statements.

Embodiments may solve or mitigate one or more of Technical Problems A to C.

## Technical Solution for Solving the Problem

### Introduction

Embodiments present a novel approach to computational discovery of site-specific phosphorylation reactions. The approach combines knowledge graphs, relational learning, and transformation of phosphorylation data using consensus motifs of kinase families. The embodiments may solve any or all of technical problems A) B) or C) as follows:

A) By adopting statistical relational learning, embodiments may solve Technical Problem A because they may take into account the whole phosphorylation network and additional context information as a comprehensive graph. This approach may utilise statistical patterns hidden in the data and leverage the presence of known specific relations to predict new phosphorylations. For example, the system may exploit long-range statistical dependencies over chains of proteins with different relation types, which none of the prior art does.

B) To solve Technical Problem B (i.e., overcoming the lack of models able to process context-dependent phosphorylation graphs), embodiments may convert phosphorylation data sets into typed binary relations. The types are based on discovery of substrate sequence motifs preferred by particular kinase families. These motifs are secondary structures grouping similarities in structure between sequences. They are extracted from phosphorylation site contexts: the amino acids (or more strictly speaking, amino acid residues) to either side of the amino acid forming the phosphorylation site. Phosphorylation site contexts are transformed into predicates that correspond to motifs having the best compatibility with the site context sequence. These predicates provide a generalisation of the site context information that may be meaningfully compared across different statements and thus support state of the art statistical relational learning.

C) Invention embodiments may generate negatives for phosphorylation statements based on the actual biology of phosphorylation, not using the standard methods that may produce erroneous results (Technical Problem C). This solution makes use of the negative conformance of substrate sequences with respect to the predicate motifs and the fact that kinases from different families tend to bind to different substrates at different sites.

### System Overview

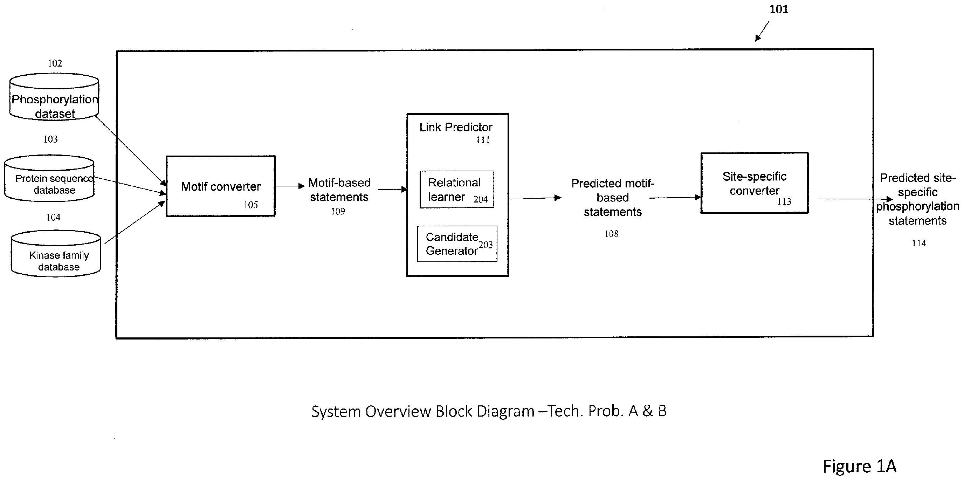

Figure 1A presents an overview of a general embodiment. The components present in the system 101 figure are as follows:

- Phosphorylation data set or network 102 is provided as an input to component 105 (the converter). It may consist of descriptions of phosphorylation interactions between a protein kinase and a protein substrate at a given location in the substrate amino acid sequence. It is assumed that each interaction may represented as a statement (or triple) of the form:

⟨*protein kinase, phosphorylation site location, protein substrate*⟩.

• Protein sequence database 103 is provided as an input for the converter to motif-based statements 105 and the converter to site-specific statements 113. At the very least, it consists of information on proteins amino acid sequences. It is assumed that each protein entry may contain at least the following information:

⟨*protein, substrate sequence*⟩,

e.g., ⟨P04637, MEEPQSDPSVEPPLSQETFS....⟩
• Kinase family database 104 is provided as an input to the motif converter 105. It consists of a mapping from all protein kinases to their corresponding kinase family. It is assumed that the mapping is represented in the form:

⟨*protein kinase, kinase family*⟩.

• The motif converter for conversion to motif-based statements 105 converts the phosphorylation network consisting of site-specific statements to generalised motif-based statements 109, as will be explained in more detail in the following. A motif is a common or consensus sequence pattern (of amino acids) relating to one or more proteins.
• Motif-based statements 109 are converted phosphorylation statements with (actionable) motif-based predicates.
• Link Predictor/Prediction Unit 111 handles the prediction of new motif-based phosphorylation statements. It uses a candidate generator 203 to create new candidates from the motif-based statements and a relational learner 204 to use the candidate statements in a learning model and to score them and thus provide a set of predicted statements which is a subset of the candidate statements. The output statements may be scored based on the level of confidence the model associates with them. Such weights may be provided alongside of the statements (e.g., in the same tuple).
• The site-specific converter 113 for conversion to Site-specific Statements converts the predicted motif-based statements to a "standard" format of site-specific phosphorylation statements which may consequently be processed by the end users (i.e., biomedical scientists) for their own purposes. The component preserves any scores generated by link predictor 111 and possibly augments them by scores specific to the conversion process. This results in the construction of predicted site specific phosphorylation statements 114.
• The predicted site specific phosphorylation statements 114 may be list(s) of statements in the form:

⟨*protein kinase, phosphorylation site location, protein substrate, [scores]* ⟩.Each statement may include a set of scores generated by Link Predictor 111 and Site-specific converter 113). These scores may be used for ranking the results (which may be naturally used for determining the statements that are most likely to correspond to actual phosphorylations).

One idea of embodiments as depicted in Figure 1A is to process the known phosphorylation data and potentially also related contextual data as a knowledge graph of motif-based statements 109, using a link prediction technology. This approach may yield previously unknown (predicted) phosphorylation statements 114.

Conversion of the site-specific (i.e., protein site-context-dependent) statements to triples with actionable predicates (i.e., links) is based on generalisations of the phosphorylation sites (as motifs). The conversions between these types of statements are realised by the two converters and may use auxiliary information on the proteins and phosphorylation sites. Embodiments may be capable of generating and scoring any statement that includes kinase and substrate present in the input data, and a site that corresponds to motifs generated from the input data. This can be used for producing a broad range of predictions on any protein present in the input data.

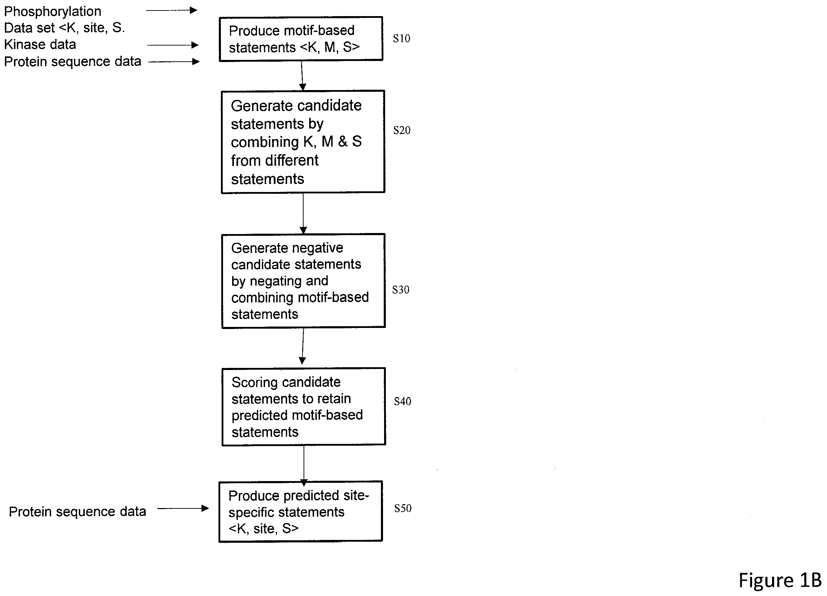

Figure 1B is a flow chart depicting the method of a general invention embodiment. In step S10, motif-based statements <K, M, S> are produced from the inputs of a phosphorylation data set which has statements of the form <K, site, S>. In order to do this the method uses kinase data indicating families which kinases are grouped into are used, as well as protein sequence data which gives the sequence for proteins. From the motif-based statements generated in step S10, candidate sequences are generated in step S20 by combining the kinases, motifs and substrates from different statements. This may be as a Cartesian product in that each kinase is combined with each motif and each substrate. In step S30 negative candidates are generated. This is by negating the motif-based statements and then use of combining, potentially using a Cartesian product. Preferred methods of negation are detailed later.

In step S40 the candidate statements are scored (also using the negative candidate statements) and only higher scoring candidate statements are retained as predicted motif-based statements.

Finally in step S50 protein sequence data is input to allow the motif-based statements to be converted to predicted site-specific statements in the form <K, site, S>.

**Example 1**

*Examples of existing knowledge on phosphorylations (i.e., site-specific phosphorylation statements) are: ⟨P04049, S99, Q92934⟩, ⟨P23588, S497, P04049⟩, ⟨P04049, S259, P04049⟩. In these statements, the first element corresponds to a kinase protein (the phosphorylation "agent") and the third element corresponds to a substrate protein (the phosphorylation "patient"). The second element corresponds to a phosphorylation site in the substrate amino acid sequence. The site identifier is composed of the amino acid residue (indicated by the letter) and the position of the residue in the substrate sequence (indicated by the number).*

*P04049 in the statements above refers to RAF1, an important human regulatory kinase involved in many pathways related to cancer. The examples show how a kinase may also act as a substrate and thus participate in so called phosphorylation cascades. Kinases may also phosphorylate themselves in feedback loops.*

*It is assumed that a biologist wants to find out whether or not RAF1 / P04049 could phosphorylate the substrate Q92915 which refers to Fibroblast growth factor 14, a not so well understood protein that is suspected to be involved in nervous system development and possibly also in related malignancies.*

*The existing techniques cannot predict anything if provided with just a list of statements (i.e., knowledge graph) like the ones above. They typically require custom data sets built from protein and phosphorylation site databases, manually processed features and positive / negative example batches.*

*Embodiments may, however, process the data from publicly available resources purely automatically and come up with a prediction such as ⟨P04049, T190, Q92915⟩, which may consequently be processed by the biologist in a wet lab that is a physical, real-life laboratory as opposed to a simulation or mathematical exploration. This illustrates how embodiments target Technical Problem A differently from the prior art.*

**Example 2**

*The link prediction unit 111, however, requires statements with actionable predicates, not the phosphorylation site information in order to be able to produce meaningful predictions. Using prior art in link prediction with statements like ⟨P04049, S99, Q92934⟩, gives meaningless results since S99 in protein Q92934 generally means something totally different from S99 in any other protein. This is due to the fact that it is just a pointer to a specific place in a substrate protein sequence and not something that may be generalised across different proteins (a key requirement for an actionable predicate in the scope of link prediction).*

*The inventors have realised that the statements therefore need to be converted before and after link prediction to produce meaningful results. An example of a statement converted to an internal format that may be meaningfully processed by link prediction is ⟨P04049, TKL$^2$, Q92934⟩ where TKL$^2$ refers to a predicate that generalises information on multiple phosphorylation sites and therefore may be compared across different substrate proteins.*

*This addresses Technical Problem B and is handled by Converters 105, 113 which connect between site-specific and generalised (motif-based) in Figure 1A. Examples of more technical details in the conversion process will be given later, after the description of the specific components.*

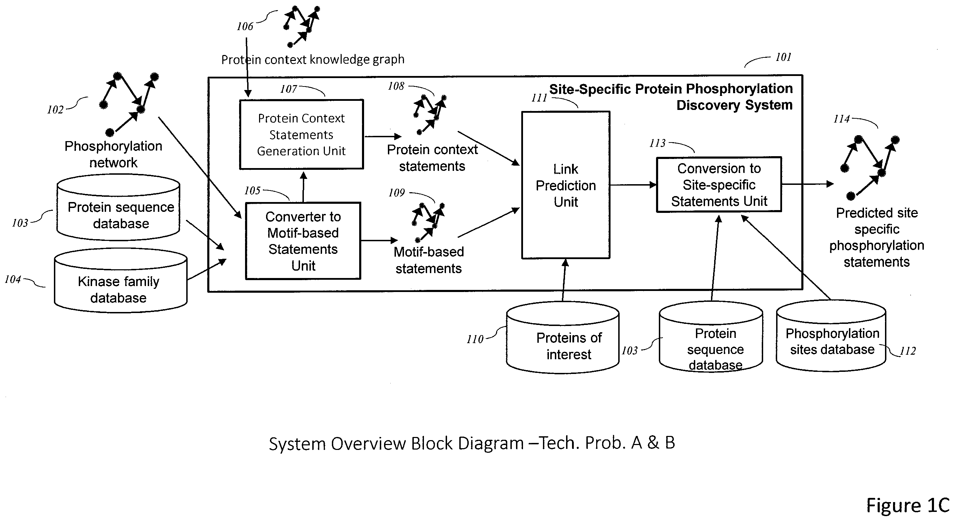

Figure 1C is an overview of a more specific embodiment. In addition to the parts already described for Figure 1A above, Figure 1C includes the following components:

- Protein context knowledge graph 106 is provided as an input to component 107 (the protein context statement generator). It consists of statements related to the protein kinases and protein substrates such as additional protein-protein interactions. It is assumed the knowledge graph is represented in the form of a tuple:

    ⟨*subject, relation, object*⟩
    indicating that *subject* and *object* entities (proteins) hold a relationship *relation (such as the two bind together).*

- Protein Context Statements Generation Unit 107 extracts additional statements from protein context knowledge graph on the proteins kinases and substrates part of the motif-based statements. This leads to the construction of protein context statements for the proteins for which there are site-specific statements, represented in the form of a tuple:

    ⟨*subject, relation, object*⟩

- Protein context statements 108 selected triples from protein context knowledge graph 106 expressing additional contextual information on protein kinases and protein substrates coming from the motif-based statements 109.

- Proteins of interest 110 Kinases or substrate proteins of interest may be provided as an input to component 111 (the link predictor). This input may consist of a list of one or more proteins for which the user would like to have predictions (e.g., information on which substrates given kinases possibly phosphorylate at which specific site). If this input is not provided, the predictions may be computed on a set of all possible phosphorylations induced by the kinases and substrates present in the input data.

- Phosphorylation sites database 112 may be provided as an input for component 113. It consists of information on all potential phosphorylation sites of protein substrates. It is assumed that each protein entry contains at least the following information:

> ⟨*protein, list of phosphorylation sites*⟩,
> e.g., ⟨*P04637, [S106, T523, ...]*⟩.

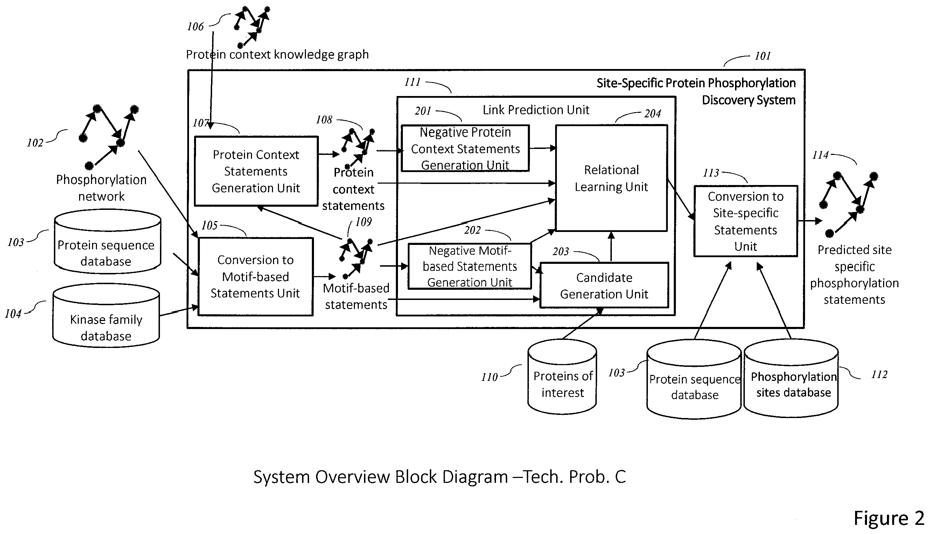

The approach of embodiments solving Technical Problem C is illustrated in Figure 2 (which gives more details of components that may be used in the Link Predictor 111 of Figure 1). Components present in the figure are as follows:

- **Negative protein context statements generation unit 201 generates negative** statements based on any positive protein context statements 108, using a known subject and object corruption technique in link prediction state of the art (see, for example Maximilian Nickel et al. "A review of relational machine learning for knowledge graphs" In: arXiv preprint arXiv: 1503.00759 (2015)).
- **Negative motif-based statements generation unit 202** generates negative statements from the motif-based positive phosphorylation statements 109 using a technique according to an embodiment.
- **Candidate generation unit 203** generates a set of candidate motif-based statements related to the proteins of interest 110 (or all proteins from the kinases and substrates present in the input data), and a corresponding subset of negative candidate statements. These statements may be used for generating ranked phosphorylation predictions by relational learner 204, once a model has been learned as explained below.
- **Relational learning unit 204** is an off-the-shelf statistical relational learning model that learns from the enriched knowledge graph (for example positive and negative motif-based statements as well as optional positive and negative protein context statements), and uses the learned model for *link prediction*, i.e., for computing scores of the candidate statements generated by candidate generator 203. The scores may be used later on for determining which of the statements are most likely to be actual phosphorylations.

The Link Predictor/Prediction Unit (111 macro-component from Figure 1) is detailed by Figure 2. There are multiple possible solutions for predicting links in graph-like knowledge bases (see for example Matt Gardner and Tom M Mitchell. "Efficient and Expressive Knowledge Base Completion Using Subgraph Feature Extraction". In: EMNLP. 2015, pp. 1488-149 and Robert West et al. "Knowledge Base Completion via Search-based Question Answering". In: Proceedings of the 23rd International Conference on World Wide Web. WWW '14. Seoul, Korea. ACM, 2014, pp. 515-526, or the approaches reviewed in Maximilian Nickel et al. "A review of relational machine learning for knowledge graphs". In: arXiv preprint arXiv: 1503.00759 (2015)). Any off-the-shelf relational learning algorithm may be used for implementing the Link Predictor 111. However, most easily applicable, accurate and tractable solutions require examples of negative statements (see Nickel et al) for both the phosphorylation and for any protein context statements 108, 109. For the protein context statements 108 that are processed by the model as is (i.e., without conversion to a different format), one strategy is to use a standard technique used in the field of link prediction in negative protein context generator 201. However, the motif-based statements 109 require a specific method 202 which is the core of an advantageous solution to Technical Problem C.

The relational learning unit 204 takes the positive and negative phosphorylation and any protein context statements as the primary input and learns a predictive model. The model may then be used to produce ranked phosphorylation statements related to an externally defined (e.g., by experts) list of proteins of interest 110. The ranked statements produced by the model are converted to the site-specific predicted phosphorylation statements 114 (by converter 113) which are the final product of the system.

## Example 3

*The converted positive statement <P04049, TKL$^2$, Q92934> from the previous example needs to be accompanied by corresponding negative statements in order for some relational learning units to work. However, the standard corruption-based technique (see Maximilian Nickel et al) applied in traditional link prediction approaches may be unsafe in this particular case.*

*This is due to the indirect nature of the predicate* TKL$^2$*. Using a standard approach common in the prior art will result in association of corrupted kinases or substrates with the complement of the positive statement: statements <X,*

*TKL² Q9293> or <P04049, TKL² Y>, where X and Y never occur with the <TKL², Q9293> and <P04049, TKL²> (predicate, object) and (subject, predicate) pairs, respectively.*

*These corrupted statements may still be positive after the reverse conversion to the site-specific format 113. For instance, a corruption <P04049, TKL², Q2750> corresponds to the site-specific statement ⟨P04049, S222, Q2750⟩. However, this statement represents an actual known phosphorylation, and therefore considering its counterpart <P04049, TKL², Q02750> would result in flawing the training of the link prediction model.*

*Therefore another, more biologically secure method is proposed here for generating the negatives corresponding to the statements converted by the converter into motif-based statements 105. This approach utilises a process based on the same biological assumptions as the conversion process (explained in detail in further examples later), which addresses Technical Problem C better is possible with the prior art.*

The following sections provide details of the particular components present in the high-level Figures 1 and 2. Tables are referred to in the examples. A kinase and a substrate in the phosphorylation network 102 are denoted as "Kin" + <*number*> and "Sub" + <*number*> respectively. A protein which appears in the protein context knowledge graph 106 but does not appear in the phosphorylation network 102 is denoted as "Prot" + <*number*>. Note that these notations are for understandability and simplicity although a kinase and a substrate are also proteins, and some proteins may be both a kinase and a substrate.

**Conversion to Motif-Based Statements**

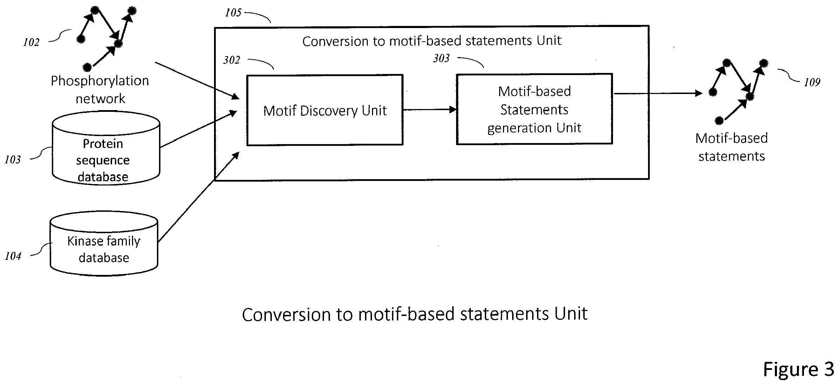

Figure 3 describes in detail the conversion to motif-based statements.

The converter 105 is a "macro component" composed of the following specific components:

- **Motif Discovery Unit 302** aims at finding position-specific scoring matrices (used as motifs) for each kinase family.
- **Motif-based Statement Generation Unit 303** converts the site-specific phosphorylation statements to (positive) motif-based statements 109 using the best conforming motif from the same kinase family.

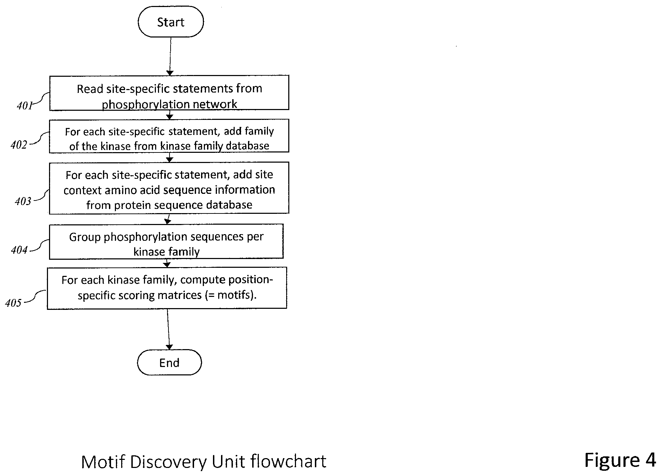

The flowchart in Figure 4 describes how an embodiment discovers motifs from site-specific statements using steps 401 to 405 described below:

- **401.** This process starts by reading all the site-specific statements of the form:

    ⟨*protein kinase, phosphorylation site location, protein substrate*⟩ from the phosphorylation network 102.

- **402.** Then for each statement's kinase, the system looks for its corresponding kinase family in the kinase family database 104. The information as to the kinase family is added to the site-specific statement.
- **403.** Similarly, the context interims of (left-hand-side/right-hand-side) amino acids to either side in the protein sequence (of the phosphorylation site) is added for each site-specific statement using information from the protein sequence database 103. This results in a "context sequence" of amino acids.
- **404.** The next step groups context sequences by kinase family resulting in a mapping from kinase family to a list of phosphorylation site context sequences of protein substrates phosphorylated by kinases of that family.
- **405.** The last step is to compute position-specific scoring matrices of the motifs. The positional scoring matrix is a representation of a motif and these two terms are used interchangeably in the rest of the text. For each possible amino acid position in a motif sequence (of, say 7 to 15 positions to either side) centred on the phosphorylation site), the motif provides a score (e.g., the log likelihood score) for each amino acid at that position. More details for a position weight matrix may be found at https://en.wikipedia.org/wiki/Position_weight_matrix. An off-the-shelf bioinformatics tool may be used to compute the motifs. As a result of this process, a list of motifs is generated for each kinase family. Note that all the motifs for a kinase family may be processed at once and that a number of batches created by dividing the motifs may be processed separately (results being merged). For creating batches, the motifs may be divided by a prefixed size of a batch, may be divided by the protein (one alphabet using the single letter code) of the phosphorylation site, and or both may be combined.

**Example 4. Motif Discovery Unit**

*Step 401 reads the site-specific statements from the phosphorylation network as*

**[Table 1] Phosphorylation Table**

| Kinase | Phosphorylation site | Substrate |
|---|---|---|
| Kin00 | S99 | Sub00 |
| Kin00 | S619 | Sub01 |
| Kin01 | S211 | Sub02 |
| Kin02 | T62 | Sub03 |
| ... | ... | ... |

*and creates the Augmented Phosphorylation Table 2 with a statement ID (abbreviated as "stmt. id"), an empty kinase family, and an empty context sequence (to be filled later). As shown in Table 1, a kinase may phosphorylate more than one substrate in general. A kinase may phosphorylate more than one site on the same substrate in general (not illustrated). For example, for the first record in Table 1, the first record in Table 2 is created with the statement id "000"*

**[Table 2] Augmented Phosphorylation Table**

| Stmt. id | Kinase | Kinase family | Phosphorylation site | Substrate | Context sequence |
|---|---|---|---|---|---|
| 000 | Kin00 | | S99 | Sub00 | |
| 001 | Kin00 | | S619 | Sub01 | |
| 002 | Kin01 | | S211 | Sub02 | |
| 003 | Kin02 | | T62 | Sub03 | |
| ... | ... | | ... | ... | |

Step 402 refers to Kinase Family Table 3 in the kinase family database (104), and for each record in Augmented Phosphorylation Table 2, fills into the "kinase family" cell a kinase family corresponding to the kinase in the "kinase" cell.

**[Table 3] Kinase Family Table**

| Kinase | Kinase family |
|---|---|
| Kin00 | TKL |
| Kin01 | TKL |
| Kin02 | AGC |
| ... | ... |

For example, for the first record (with the statement id "000") in Table 2, because the kinase is "Kin00" and its family is "TKL" derived from Table 3, "TKL" is filled into the "kinase family" cell. Finally, Augmented Phosphorylation Table 2 becomes Augmented Phosphorylation Table 4.

**[Table 4] Augmented Phosphorylation Table**

| Stmt. id | Kinase | Kinase family | Phosphorylation site | Substrate | Context sequence |
|---|---|---|---|---|---|
| 000 | Kin00 | TKL | S99 | Sub00 | |
| 001 | Kin00 | TKL | S619 | Sub01 | |
| 002 | Kin01 | TKL | S211 | Sub02 | |
| 003 | Kin02 | AGC | T62 | Sub03 | |
| ... | ... | ... | ... | ... | |

Step 403 refers to Protein Sequence Table 5 in the protein sequence database (103), and for each record in Augmented Phosphorylation Table 4, fills the "context sequence".

### [Table 5] Protein Sequence Table

| Protein | Sequence |
|---|---|
| Sub00 | MFQIPEFEPS EQEDSSSAER GLGPSPAGDG PSGSGKHHRQ APGLLWDASH QQEQPTSSSH HGGAGAVEIR SRHSSYPAGT EDDEGMGEEP SPFRGRSRSA PPNLWAAQRY GRELRRMSDE FVDSFKKGLP RPKSAGTATQ MRQSSSWTRV FQSWWDRNLG RGSSAPSQ |
| ... | ... |

Processing of the first record (with the statement id "000") in Table 4 is shown as a concrete example as follows. Because the substrate is "Sub00", the record with the protein "Sub00" is looked up and the sequence "MFQIPEFEPS EQEDSSSAER GLGPSPAGDG PSGSGKHHRQ APGLLWDASH QQEQPTSSSH HGGAGAVEIR SRHSSYPAGT EDDEGMGEEP SPFRGRSRSA PPNLWAAQRY GRELRRMSDE FVDSFKKGLP RPKSAGTATQ MRQSSSWTRV FQSWWDRNLG RGSSAPSQ" (a space is inserted by every 10 characters for visibility) is obtained. Because the phosphorylation site is "S99", the site context around the 99th character "S" is extracted. Any length may be possible, but here the left 7 characters and the right 7 characters (and the 99th character itself) are defined as a context (relevant part of the sequence). In this example, the sequence from the 92nd character and the 106th character, that is, "PFRGRSRSAPPNLWA" (underlined), is extracted and filled into the "context sequence" cell. Finally, Augmented Phosphorylation Table 4 becomes Augmented Phosphorylation Table 6.

### [Table 6] Augmented Phosphorylation Table

| Stmt. id | Kinase | Kinase family | Phosphorylation site | Substrate | Context sequence |
|---|---|---|---|---|---|
| 000 | Kin00 | TKL | S99 | Sub00 | PFRGRSRSAPPNLWA |
| 001 | Kin00 | TKL | S619 | Sub01 | SLPKINRSASEPSLH |
| 002 | Kin01 | TKL | S619 | Sub02 | SLPKINRSASEPSLH |
| 003 | Kin02 | AGC | T62 | Sub03 | RRPESFTTPEGPKPR |
| ... | ... | ... | ... | ... | ... |

Step 404 groups context sequences by each kinase family and creates Context Sequences Table 7.

### [Table 7] Context sequences Table

| Kinase family | Context sequences |
|---|---|
| TKL | {PFRGRSRSAPPNLWA, SLPKINRSASEPSLH, ... } |
| AGC | {RRPESFTTPEGPKPR, ...} |
| ... | ... |

For example, for the kinase family "TKL", the context sequences in the first three records (with the statement ids "000", "001", and "002") are grouped. Note that there may be more records in the whole table though there are only three records in the exemplified records. As a result, "FPFRGRSRSAPPNLWA, SLPKINRSASEPSLH, ...}" is obtained for "TKL" and is stored in Context Sequences Table 7 as shown in the first record.

Step 405 computes motifs for each kinase family and creates Motif Table 8.

**[Table 8] Motif Table**

| Kinase family | Modif id | | A | C | D | E | F | G | H | I | K | L | M | N | P | Q | R | S | T | V | W | Y |
|---|---|---|---|---|---|---|---|---|---|---|---|---|---|---|---|---|---|---|---|---|---|---|---|
| | | | | | | | | | | | | | | | | | | | | | | | PSSM |
| TKL | TKL0 | 1 | 1 | 66 | 4 | -22 | 54 | 32 | 28 | -137 | 89 | -47 | -45 | 57 | 37 | 59 | -29 | -72 | -115 | 10 | 45 | 30 |
| | | 2 | 1 | 66 | -16 | 6 | 29 | -4 | -2 | 22 | 48 | 8 | -134 | 44 | 18 | 10 | 35 | -29 | -64 | -58 | 45 | -39 |
| | | 3 | -14 | -124 | 3 | 49 | -96 | -20 | 72 | 46 | -68 | -24 | 29 | -25 | 45 | -7 | 93 | -55 | -52 | 22 | -379 | -2 |
| | | 4 | 12 | 24 | 36 | -61 | 74 | 19 | 51 | -11 | 46 | 5 | 79 | 55 | -8 | 46 | -92 | -65 | -53 | -41 | -390 | -3 |
| | | 5 | 34 | -476 | -42 | -25 | -4 | 30 | -42 | 46 | 10 | 14 | 47 | -5 | 36 | -45 | 44 | -39 | -53 | 8 | -390 | -3 |
| | | 6 | 21 | 63 | 34 | 2 | 25 | -40 | 24 | 18 | 18 | -40 | 46 | -52 | -10 | 6 | 69 | 0 | -87 | -90 | -429 | 73 |
| | | 7 | 10 | 63 | 34 | -12 | -6 | 57 | -98 | -32 | 18 | 12 | 46 | 41 | -38 | 33 | -52 | -20 | -44 | 6 | 42 | -100 |
| | | 8 | -551 | -637 | -885 | -952 | -686 | -693 | -832 | -903 | -988 | -908 | -896 | -820 | -873 | -902 | -925 | 207 | 238 | -770 | -642 | 76 |
| | | 9 | -16 | -33 | -19 | 27 | -44 | 18 | 50 | -30 | -12 | -5 | 28 | 28 | 35 | 34 | -121 | -5 | -30 | 20 | -402 | 52 |
| | | 10 | 23 | -127 | -19 | 27 | -44 | 6 | -5 | 45 | 10 | -26 | -88 | -50 | 53 | -26 | 53 | 7 | -102 | 7 | 43 | -4 |
| | | 11 | 63 | 65 | -41 | 5 | 109 | -6 | 27 | -10 | -108 | -24 | -17 | -5 | -20 | 23 | -15 | -31 | -66 | 74 | 140 | 75 |
| | | 12 | 26 | -118 | 52 | 7 | -92 | -51 | -1 | 8 | 40 | 41 | -81 | 15 | -4 | -4 | 1 | -28 | -49 | 11 | 198 | -38 |
| | | 13 | -26 | -27 | 6 | 8 | 0 | 22 | 53 | -45 | 33 | 26 | -80 | -1 | -4 | -88 | 26 | -51 | 22 | 67 | -342 | -88 |
| | | 14 | -42 | 98 | -14 | 31 | -38 | -3 | -329 | 70 | 14 | 74 | 32 | -71 | -135 | 60 | 2 | -69 | -4 | 36 | 46 | -168 |
| | | 15 | 47 | -117 | 88 | 99 | -38 | 10 | 29 | -7 | -63 | 34 | 50 | 58 | -4 | 12 | -12 | -115 | -62 | -56 | -342 | -168 |
| TKL | TKL1 | ... | | | | | | | | | | | | | | | | | | | | |
| TKL | ... | ... | | | | | | | | | | | | | | | | | | | | |
| AGC | AGC0 | ... | | | | | | | | | | | | | | | | | | | | |
| ... | ... | ... | | | | | | | | | | | | | | | | | | | | |

*If no sequences are present for a family, no motifs are generated. Here a motif is represented as a position-specific matrix (PSSM) but any other form may be adopted as long as motifs are defined and the score between each motif and a context sequence may be calculated. In this example, MEME (see http://meme-suite.org) is used. MEME is a tool which inputs a set of sequences of the same length (in this usage according to embodiments; generally the input of MEME may be a set of sequences of various lengths) and outputs a set of motifs. In essence, the algorithm tries to find a pattern in the frequencies of occurrences of specific amino acids in particular locations in the input sequences in order to give a generalisation of the batch of sequences in the form of a convenient motif representation. The EM algorithm is used to find a reasonably optimal pattern of frequency distributions by trying to find a solution as far away from random noise as possible. An authoritative description from the tool authors can be found in the following paper and in related references: https://academic.oup.com/nar/article/37/suppl_2/W202/1135092/MEME-Suite-tools-for-motif-discovery-and-searching.*

*Each motif is a position-specific matrix. A position-specific matrix is a matrix of 20 columns (for 20 letters of the protein alphabet) and rows. The number of rows is equal to the length of the context sequence (again in this usage; generally the rows of the matrix may be of arbitrary various lengths), that is, 15 here (7 to either side of the phosphorylation site). Each cell represents a score. The meaning of the scores and the usage of the matrix will be shown in a later example.*

*In Motif Table 8, for example, for "TKL", several motifs are generated by MEME, or another suitable tool.*

*The motifs are discovered by MEME. However, the number and granularity of the motifs per each family are influenced by two hyperparameters of the motif extracting tool (at least in the current embodiment): the size of the context window and the number of sequences in randomised samples that are processed by MEME at once.*

*The number of motifs discovered per family cannot be directly set by the user - the actual number depends on the data, and also on the values of the context window and the number of sequences in randomised samples, but these are reflected only very indirectly. There is no user defined confidence threshold - the MEME algorithm is based on expectation maximisation and this statistical method finds the optimal motifs without external parameters once a sufficiently high number of good motifs is specified (as in the current embodiment, to make sure all optimal motifs found are included). 10 may be used in the current embodiment, and typically there may be only up to 5 motifs found for any combination of hyperparameters used to run the experiments.*

*Each motif is attached a motif id such as "TKL0" and "TKL1". In the "PSSM" row, a PSSM is filled for a motif id (only the PSSM for "TKL0" is shown).*

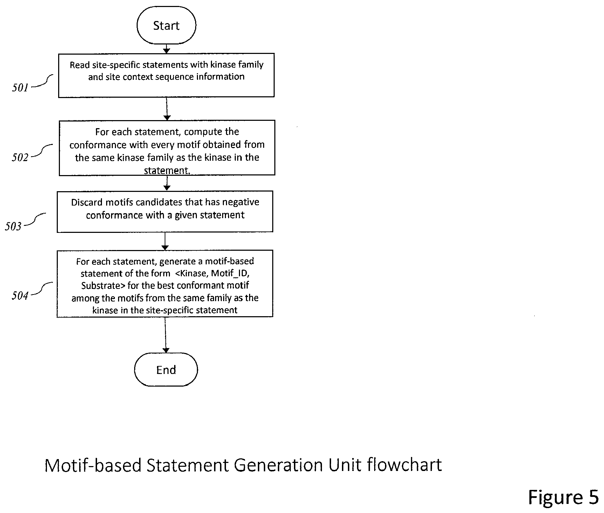

The flowchart in Figure 5 describes how site specific statements are converted in motif-based statements:

- **501.** This process starts by reading all the site-specific statements enriched with the kinase family and phosphorylation site context sequence information. The resulting statements are in the form:

  ⟨*kinase, kinase family, substrate, phosphorylation site context sequence*⟩.

- **502.** Then for each statement's context sequence, the system computes the conformance between the given context sequence and every motif discovered for the family of the kinase in the statement. A conformance score is obtained using the following function:

$$score(S, M) = \sum_{i \in \{1, ..., |S|\}} M_{i, id(S_i)},$$

  where *S, M* is the sequence (represented as a linear chain, i.e., vector) and the motif scoring matrix, respectively. *id* is a function mapping the letters of the protein alphabet to the corresponding column indices in the motif matrix. For each pair of ⟨*statement, phosphorylation motif of the same kinase family*⟩ a conformance score is given.

- **503.** Only pairs of
  ⟨ *statement, phosphorylation motif of the same kinase family*⟩
  with a positive conformance score are kept. Negative conformance means there is no conformance and therefore the motif cannot be considered as representing the statement context sequence.

- **504.** For each site-specific statement, a motif-based statement is created by selecting the best performing (in relation to the conformance score) motif. This process therefore generates a list of positive (positive in the sense that they actually occur in real life) motif-based statements of the form:

  ⟨*protein kinase, motif, protein substrate*⟩.

These statements are not context dependent anymore, as the motif-based predicate generalises the context information in a way that may be compared across multiple phosphorylation statements (which is a necessary condition for consequent link prediction).

**Example 5. Motif-Based Statement Generation Unit**

*Step 501 reads Augmented Phosphorylation Table 6 created in the previous example.*

**[Table 6] (reprinted) Augmented Phosphorylation Table**

| Stmt. id | Kinase | Kinase family | Phosphorylation site | Substrate | Context sequence |
|---|---|---|---|---|---|
| 000 | Kin00 | TKL | S99 | Sub00 | PFRGRSRSAPPNLWA |
| 001 | Kin00 | TKL | S619 | Sub01 | SLPKINRSASEPSLH |
| 002 | Kin01 | TKL | S619 | Sub02 | SLPKINRSASEPSLH |
| 003 | Kin02 | AGC | T62 | Sub03 | RRPESFTTPEGPKPR |
| ... | ... | ... | ... | ... | ... |

*For each record in Table 6 and each motif of the record's kinase family in Motif Table 8 created in the previous example, Step 502 computes a conformance score and stores a corresponding record in Conformance Score Table 9.*

**[Table 9] Conformance Score Table**

| Stmt. id | Motif id | Conformance score |
|---|---|---|
| 000 | TKL0 | 528 |
| 000 | TKL1 | -35 |
| 000 | TKL2 | 187 |
| ... | ... | ... |

*Concretely, suppose the first record with the statement id "000" in Table 6 is processed. Because the kinase family is "TKL", the motifs whose kinase family is "TKL" in Table 8 are processed. Here the calculation details are explained for "TKL0".*

*The meaning of a PSSM corresponding to a kinase family is as follows. First, the meaning of a score in a cell:*

- *If a score in a column* i *and a row* X *is greater than 0, then the likelihood of an amino acid* X *occurring on the position* i *in a sequence corresponding to the kinase family in question is greater than it would be in a purely random case (i.e., the kinase family tends to prefer the amino acid at that position).*
- *If a score in a column* i *and a row* X *is equal to 0, then the occurrence of an amino acid* X *on the position* i *cannot be distinguished from a random case (i.e., the kinase family does not have any preference for the amino acid at that position).*
- *If a score in a column* i *and a row* X *is less than 0, then the likelihood of an amino acid* X *occurring on the position* i *in a sequence corresponding to the kinase family in question is lower than it would be in a purely random case (i.e., the kinase family tends to stay away from the amino acid at that position).*

*For example, some cells of the PSSM for the motif id "TKL0" mean:*

- *The score in the column "3" and the row "R" is 93, which is greater than 0. Therefore, if the third character of a sequence is "R", the sequence is positively associated with the motif in its third letter.*
- *The score in the column "6" and the row "S" is 0. Therefore, if the sixth character of a sequence is "S", the association of the sequence at the sixth letter with the motif cannot be determined as it is about as likely as random.*
- *The score in the column "9" and the row "A" is -16, which is lower than 0. Therefore, if the ninth character of a sequence is "A", the sequence is disassociated with the motif at the ninth letter.*

*A conformance score of a sequence is calculated by summing up the scores of all the positions in the sequence - this corresponds to the likelihood of the sequence's conformance to the motif across all the sequence letters. In the*

*current example, the input sequence is "PFRGRSRSAPPNLWA" (the one for the statement id "000"). The conformance score is calculated by summing up the 15 shaded scores in Table 10.*

**[Table 10] PSSM**

|    | A | C | D | E | F | G | H | I | K | L | M | N | P | Q | R | S | T | V | W | Y |
|----|---|---|---|---|---|---|---|---|---|---|---|---|---|---|---|---|---|---|---|---|
| 1 | 1 | 66 | 4 | -22 | 54 | 32 | 28 | -137 | 89 | -47 | -45 | 57 | 37 | 59 | -29 | -72 | -115 | 10 | 45 | 30 |
| 2 | 1 | 66 | -16 | 6 | 29 | -4 | -2 | 22 | 48 | 8 | -134 | 44 | 18 | 10 | 35 | -29 | -64 | -58 | 45 | -39 |
| 3 | -14 | -124 | 3 | 49 | -96 | -20 | 72 | 46 | -68 | -24 | 29 | -25 | 45 | -7 | 93 | -55 | -52 | 22 | -379 | -2 |
| 4 | 12 | 24 | 36 | -61 | 74 | 19 | 51 | -11 | 46 | 5 | 79 | 55 | -8 | 46 | -92 | -65 | -53 | -41 | -390 | -3 |
| 5 | 34 | -476 | -42 | -25 | -4 | 30 | -42 | 46 | 10 | 14 | 47 | -5 | 36 | -45 | 44 | -39 | -53 | 8 | -390 | -3 |
| 6 | 21 | 63 | 34 | 2 | 25 | -40 | 24 | 18 | 18 | -40 | 46 | -52 | -10 | 6 | 69 | 0 | -87 | -90 | -429 | 73 |
| 7 | 10 | 63 | 34 | -12 | -6 | 57 | -98 | -32 | 18 | 12 | 46 | 41 | -38 | 33 | -52 | -20 | -44 | 6 | 42 | -100 |
| 8 | -551 | -637 | -885 | -952 | -686 | -693 | -832 | -903 | -988 | -908 | -896 | -820 | -873 | -902 | -925 | 207 | 238 | -770 | -642 | 76 |
| 9 | -16 | -33 | -19 | 27 | -44 | 18 | 50 | -30 | -12 | -5 | 28 | 28 | 35 | 34 | -121 | -5 | -30 | 20 | -402 | 52 |
| 10 | 23 | -127 | -19 | 27 | -44 | 6 | -5 | 45 | 10 | -26 | -88 | -50 | 53 | -26 | 53 | 7 | -102 | 7 | 43 | -4 |
| 11 | 63 | 65 | -41 | 5 | 109 | -6 | 27 | -10 | -108 | -24 | -17 | -5 | -20 | 23 | -15 | -31 | -66 | 74 | 140 | 75 |
| 12 | 26 | -118 | 52 | 7 | -92 | -51 | -1 | 8 | 40 | 41 | -81 | 15 | -4 | -4 | 1 | -28 | -49 | 11 | 198 | -38 |
| 13 | -26 | -27 | 6 | 8 | 0 | 22 | 53 | -45 | 33 | 26 | -80 | -1 | -4 | -88 | 26 | -51 | 22 | 67 | -342 | -88 |
| 14 | -42 | 98 | -14 | 31 | -38 | -3 | -329 | 70 | 14 | 74 | 32 | -71 | -135 | 60 | 2 | -69 | -4 | 36 | 46 | -168 |
| 15 | 47 | -117 | 88 | 99 | -38 | 10 | 29 | -7 | -63 | 34 | 50 | 58 | -4 | 12 | -12 | -115 | -62 | -56 | -342 | -168 |

*For example, since the first character is "P", the cell value "37" in the column "1" and the row "P" is shaded. Similarly, since the second character is "F", the cell value "29" in the column "2" and the row "F" is shaded. By calculation of "37 + 29 + 93 + 19 + 44 + 0 - 52 + 207 - 16 + 53 - 20 + 15 + 26 + 46 + 47", the conformance score is obtained as 528. This score is stored in Table 9 as shown previously.*

*Similarly, suppose other conformance scores are calculated as shown in Table 9. That is, the conformance score for the statement id "000" and the motif id "TKL1" is -35, and the one for "000" and "TKL2" is 187.*

*Step 503 discards records with a negative conformance score in Conformance Score Table 9. In the current example, the second record has the negative conformance score "-35". As a result of discard, Conformance Score Table 9 becomes Conformance Score Table 11. Here the threshold for discard was zero but any arbitrary value may be used or with a specified value N the top N may be selected.*

**[Table 11]**

| Conformance Score Table | | |
|---|---|---|
| **Stmt. id** | **Motif id** | **Conformance score** |
| 000 | TKL0 | 528 |
| 000 | TKL2 | 187 |
| ... | ... | ... |

*For each statement id in Augmented Phosphorylation Table 6, Step 504 generates a ⟨Kinase, Motif id, Substrate⟩ statement for the motif with the best conformance score for the statement id, and stores it in Positive Motif-based Statement Table 12.*

**[Table 12]**

| Positive Motif-based Statement Table | | |
|---|---|---|
| **Subject [Kinase]** | **Predicate [Motif id]** | **Object [Substrate]** |
| Kin00 | TKL0 | Sub00 |

(continued)

| Positive Motif-based Statement Table | | |
|---|---|---|
| Subject [Kinase] | Predicate [Motif id] | Object [Substrate] |
| ... | ... | ... |

*If there are no positive conformance scores, no converted statements are generated. In the current example, there are two records in Positive Motif-based Statement Table 11 for the statement id "000". Since the conformance score 528 of the first record is greater than the conformance score 187 of the second record, the former is selected, that is, the motif id "TKL0" is selected. As a result, ⟨Kin00, TKL0, Sub00⟩ is generated as shown in Positive Motif-based Statement Table 12.*

## Enriching the Knowledge Base by Protein Contextual Information

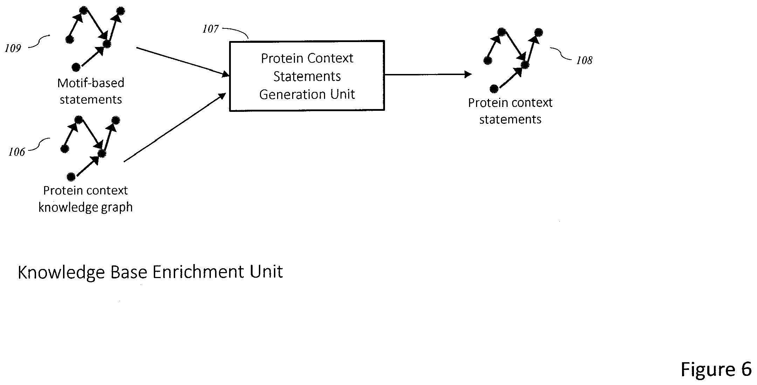

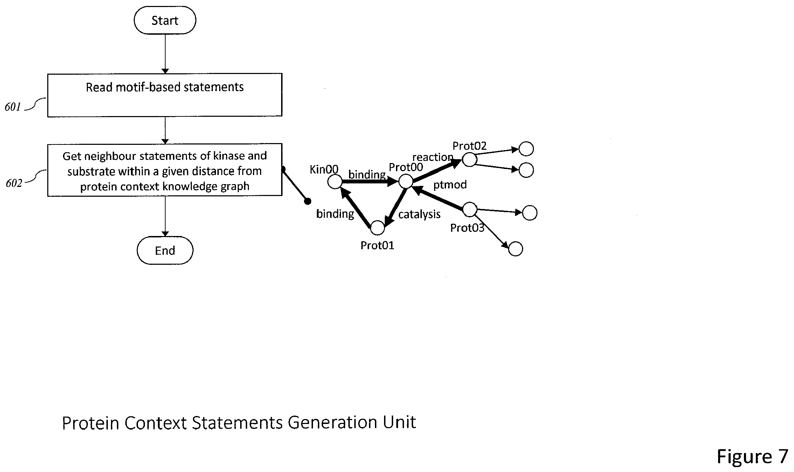

Figures 6 and 7 describe in detail the Protein Context Statement Generation Unit 107. Figure 6 includes the relevant parts already described in Figure 2. The flowchart of Figure 7 composed of the following specific steps:

- **601.** This process starts by reading all positive motif-based statements 109 of the form:

    ⟨*protein kinase, motif, protein substrate*⟩.

- **602.** Then, the system uses the protein context knowledge graph 106 to extract neighbour statements within a given distance from the protein kinases and substrates contained in the positive motif-based statements. As a result protein context statements 108 are created. The statements can be optionally used for improving the predictive capability of the model by adding more knowledge (i.e., relationships / statements) about the proteins appearing in phosphorylations. This addition is easy with the method of embodiments, while the prior art either does not allow inclusion of this extra information or allows it in a very superficial way (just for filtering predictions, not for actually computing them).

## Example 6. Protein Context Statements Generation Unit

*Step 601 reads motif-based statements in Positive Motif-based Statement Table 12.*

*Step 602 first reads Protein Context Knowledge Graph ProCSGU-1 (a simplified example is shown in Figure 7). For visibility, the graph is represented as a graph, not as a table. A node represents a protein, and an arrow represents a relation between the connecting two proteins. Step 602 then, for each protein (kinase, substrate) in Positive Motif-based Statement Table 12, retrieves neighbour statements of the protein within a given distance and stores them in a table. Suppose the distance is set as 2 and the current protein is "Kin00". For retrieving all the concerned statements, arbitrary graph search methods may be used. Since "Prot00" and "Prot01" are one step away from "Kin00" and "Prot02" and "Prot03" are two steps away from "Kin00", the five relations with a bold arrow are retrieved. The resulting table after processing only "Kin00" is Positive Protein Context Statement Table 13. For example, the "ptmod" arrow from "Prot03" to "Prot00" is stored as the record ⟨Prot03, ptmod, Prot00⟩.*

**[Table 13]**

| Positive Protein Context Statement Table | | |
|---|---|---|
| Subject | Predicate | Object |
| Kin00 | binding | Prot00 |
| Prot01 | binding | Kin00 |
| Prot00 | catalysis | Prot01 |
| Prot00 | reaction | Prot02 |
| Prot03 | ptmod | Prot00 |

**Link Prediction Unit**

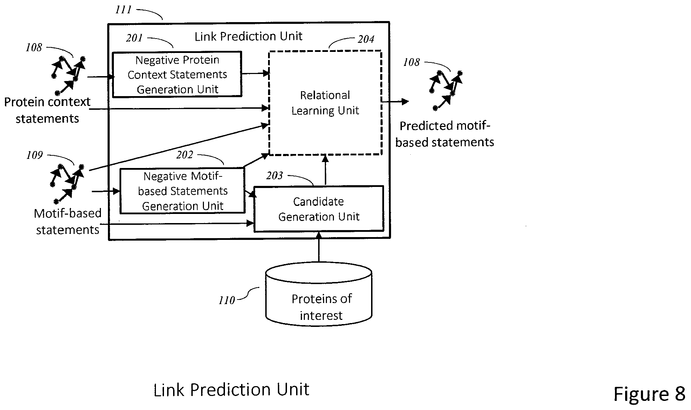

The details of the Link Prediction Unit 111 are depicted in Figure 8, which includes the relevant parts already described for Figure 2, and the output in the form of predicted motif-based statements.

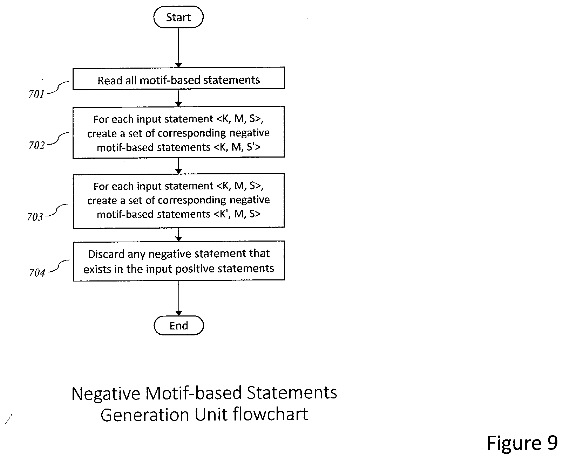

One important part of link predictor 111 in the context of some embodiments is the generation of the motif-based negative statements, detailed in Figure 9.

The flowchart of the negative motif-based statements generation unit 202 is as follows:

- **701.** This process starts by reading all positive motif-based statements 109.
- **702.** For each statement having the form:

  ⟨*protein kinase K, motif M, protein substrate S*⟩.

  the system creates a set of corresponding negative motif-based statements from substrates (substrate corruptions, or substrates which are deemed not to allow phosphorylation at the given motif and using the given kinase) that are merged into the final negative set:

$$- \{(K, M, S') \mid \forall L \in sites(S').\ score(sequence(L, S', \tfrac{|M|-1}{2}), M) < 0\},$$

  where *sites(X)* is a function returning a set of phosphorylation sites of substrate *X* for all sites *S'* at Location *L* with the score as defined. The function score is the motif-based scoring function defined in the description of motif discoverer 302 above. The *sequence* (*U, V, k*) returns the site *U* context of size *k* within *V*, i.e., a symmetric sequence of *k* amino acids on the left and right side of the site *U* in the sequence of the substrate *V*, including the site itself in the centre. We use |*M*| to indicate the width of the motif |*M*| (i.e., the number of amino acids in a sequence to be meaningfully compared against the motif). Currently embodiments use 15 and 31 which correspond to the sequence context sizes 7 and 15 as hyperparameters. A context size of 7, for example, refers to including 7 amino acids to either side of the phosphorylation site and the phosphorylation site itself. More particularly, 15 corresponds to 1 (for the amino acid which is the actual site) + 7 +7 (7 amino acids on left and right). 31 corresponds to 15 in a similar way. In essence, for a substrate that is not *S*, the context sequences are found for the same kinase and checked for conformance with Motif *M*. If this is negative, then a statement <K, M, S'> is stored as a negative statement. In the code the negatives may be generated later on the fly (rather than individually as the context sequences are found) to make the computation more efficient).

- **703**. For each statement having the form:

  ⟨*protein kinase K, motif M, protein substrate S*⟩.

  the system creates a set of corresponding negative motif-based statements from kinases (kinase corruptions or kinase which are deemed not to allow phosphorylation at the given motif and with the given substrate) and merges it to the final set by:

$$- \{(K', M, S) \mid family(K') \neq family(K)\},$$

  where *family(X)* is a function returning a family of kinase *X*.

- **704**. Any negative motif-based statement created that already exists in the positive motif-based statements is removed. The output of this process is a list of negative motif-based statements generated from the positive ones.

The rationale behind Step 702 and Step 703 is three-fold:

- First, it is important to make sure that the negatives follow the local Closed World Assumption (see Nickel et al), i.e., that the predicate is the same as in the positive triple and that a corruption occurs either at the subject (kinase) or at the object (substrate) position, but never at both. This is necessary for the relational learning unit (204) to work correctly.
- The corrupted substrates should not be compatible with the predicate motif in order for the resulting triple to be negative with a high confidence even after it is converted back to the site-specific format. A natural way of achieving that is to make sure that the score of all sites in the corruption substrate is lower than zero with respect to the predicate motif of the positive triple. This means that the corruptions will have negative likelihood of being associated with the predicate based on the actual site information.

- Similarly, the corrupted kinases should also be incompatible with the predicate motif. This may be enforced by using kinases that are from a family different than the family of the kinase in the positive statement. This will make the corrupted kinases incompatible with the positive predicate as the kinase families are mutually disjoint and the motifs are always computed from sequences within a single family.

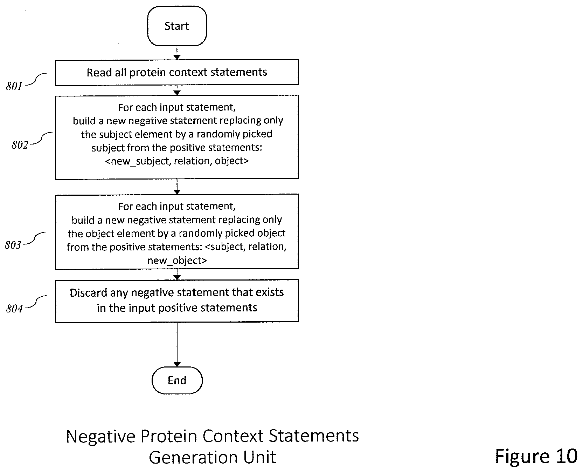

The flowchart in Figure 10 describes the process of creation of negative protein context statements 201 from positive ones.

**Negative Protein Context Statements Generation Unit**

The process is as follows:

- **801.** This process starts by reading all positive protein context statements 108 from Table 13shown previously.
- **802.** For each statement of the form:

   ⟨*subject S, relation P, object O*⟩

   the system creates new negative statements replacing only the subject element by a randomly picked subject among subjects that never occur with relation P and object O in the positives.
- **803.** For each statement of the form:

   ⟨*subject S, relation P, object O*⟩

   the system creates new negative statements replacing only the object element by a randomly picked object among objects that never occur with subject S and relation P in the positives.
- **804.** Finally, all generated negative statements present in the positive protein context statements are removed.

The protein context statements (both negative and positive) may enrich the knowledge graph and thus lead to better accuracy of the phosphorylation prediction. If the model has richer information on what may possibly be related to the kinases and substrates (for instance, via other types of protein interaction relations or publication-based co-occurrences), it may very often spot many more implicit regularities in the data and use them for better predictions.

**Example 7. Negative Motif-based Statements Generation Unit**

*Step 701 reads all the motif-based statements in Positive Motif-based Statement Table 12. Step 702 creates negative statement records for each positive statement record in Table 12 and stores them in Negative Motif-based Statement Table 15. Here the firsf record ⟨Kin00, TKL0, Sub00⟩ is used for explanation.*

**[Table 14] (enriched version of Table 6)**

| Augmented Phosphorylation Table | | | | | |
|---|---|---|---|---|---|
| Stmt. id | Kinase | Kinase family | Phosphorylation site | Substrate | Context sequence |
| 000 | Kin00 | TKL | S99 | Sub00 | PFRGRSRSAPPNLWA |
| 001 | Kin00 | TKL | S619 | Sub01 | SLPKINRSASEPSLH |
| 002 | Kin01 | TKL | S619 | Sub02 | SLPKINRSASEPSLH |
| 003 | Kin02 | ABC | T62 | Sub03 | RRPESFTTPEGPKPR |
| ... | ... | ... | ... | ... | ... |
| 100 | Kin10 | AGC | S489 | Sub10 | SDSEDDSSEFDEDDW |
| 101 | Kin11 | CAMK | S484 | Sub10 | IAVEYSDSEDDSSEF |
| 102 | Kin12 | AGC | T276 | Sub11 | LGGGRVKTWKRRWFI |
| 103 | Kin13 | CAMK | S392 | Sub11 | AARKKRISYKKKQEQ |

(continued)

| Augmented Phosphorylation Table | | | | | |
|---|---|---|---|---|---|
| **Stmt. id** | **Kinase** | **Kinase family** | **Phosphorylation site** | **Substrate** | **Context sequence** |
| ... | ... | ... | ... | ... | ... |

*Step 702 processes the following sub-steps for all the possible substrates in Augmented Phosphorylation Table 14 (or Table 12; either may be used). Table 14is an enriched version of Table 6, containing records (width the statement ids "100", "101" "102", and "103") required for explanation here.*

*In the explicitly described records in Table 14, the substrates are "sub00"; "Sub01", "Sub02", "Sub03", "Sub10", and "Sub11". In the following explanation, "Sub10" and "Sub11" are used.*

• *"Sub10"*

   ◦ *In Sub-step 702-1, all the possible context sequences (e.g., sequence around a site where phosphorylation has actually occurred for that substrate) for "Sub 10" are spotted. They are "SDSEDDSSEFDEDDW" in the record of "100" and "IAVEYSDSEDDSSEF" in the record of "101".*

   ◦ *Recall that the first record <Kin00, TKL0, Sub00> is the starting point, and only the substrate is changed. In Sub-step 702-2, for each spotted context sequence, the conformance score for the motif "TKL0" is calculated. In the same way as Step 502 with Motif Table 8, the conformance scores of "SDSEDDSSEFDEDDW" and "IAVEYSDSEDDSSEF" are calculated as -426 and -56 respectively.*

   ◦ *In Sub-step 702-3, whether all the calculated conformance scores are negative or not is checked. Since both -426 and -56 are negative, the answer is yes and a negative statement is generated whose kinase and motif id are the same as in the positive statement and whose substrate is the concerned substrate. Concretely expressed here, the kinase and motifs are "Kin00" and "TKL0" in the first record in Table 12, and the substrate is "Sub10". As a result, the first record in Negative Motif-based Statement Table 15 is stored.*

**[Table 15]**

| Negative Motif-based Statement Table | | |
|---|---|---|
| **Subject (Kinase)** | **Predicate [Motifid]** | **Object [Substrate]** |
| Kin00 | TKL0 | Sub10 |
| Kin02 | TKL0 | Sub00 |
| ... | ... | ... |

• *"Sub11"*

   ◦ *In Sub-step 702-1, all the possible context sequences for "Sub11" are spotted. They are "LGGGRVKTWKRRWFI" in the record of "102" and "AARKKRISVKKKQEQ" in the record of "103".*

   ◦ *In Sub-step 702-2, for each spotted context sequence, the conformance score for the motif "TKL0" is calculated. In the same way as Step 502 with Motif Table 8, the conformance scores of "LGGGRVKTWKRRWFI" and "AARKKRISVKKKQEQ" are calculated as -635 and 312 respectively.*

   ◦ *In Sub-step 702-3, whether all the calculated conformance scores are negative or not is checked. Since 312 is positive, the answer is no and no negative statement is stored.*

*Step 703 also creates negative statement records for each positive statement record in Table 12 and stores them in Negative Motif-based Statement Table 15. Here the first record ⟨Kin00, TKL0, Sub00⟩ is used for explanation.*

*Step 703 processes the following sub-steps for all the possible kinases in Kinase Family Table 3 (or Augmented Phosphorylation Table 14; whichever may be used). In the explicitly described records in Table 3, the kinases are "Kin00", "Kin01", and "Kin02". In the following explanation, "Kin01" and "Kin02" are used.*

*For "Kin01", whether "Kin01" and the positive statement's kinase "Kin00" belong to a same kinase family is checked by referring to Kinase Family Table 3. Since both belong to "TKL", the answer is yes and no negative statement is stored.*

*For "Kin02", whether "Kin02" and the positive statement's kinase "Kin00" belong to a same kinase family is*

*checked by referring to Kinase Family Table 3. Since the former belongs to "AGC" and the latter belongs to "TKL", the answer is no and a negative statement is stored whose motif id and substrate are the same as in the positive statement and whose kinase is the concerned kinase. Concretely, the motifs and substrate are "TKL0" and "Sub00" in the first record in Table 12, and the kinase is "Kin02". As a result, the second record in Negative Motif-based Statement Table 15 is stored.*

*Step 704 discards from Negative Motif-based Statement Table 15 all the records appearing in Table 12. In the current example, there is no such record and Table 15 is kept as it is.*

**Example 8. Negative Protein Context Statements Generation Unit**

*Step 801 reads all the (positive) protein context statements in Positive Protein Context Statement Table 13.*

*Step 802 creates negative protein context statements for each positive protein context statement and stores them in Negative Protein Context Statement Table 16.*

**[Table 16]**

| Negative Protein Context Statement Table | | |
|---|---|---|
| **Subject** | **Predicate** | **Object** |
| Prot00 | binding | Prot00 |
| prot01 | binding | Prot00 |
| Kin00 | binding | Kin00 |
| Kin00 | binding | Prot01 |
| Kin00 | binding | Frot02 |
| ... | ... | ... |

*Here the first record ⟨Kin00, binding, Prot00⟩ is used for explanation. This step first collects all the possible subjects for each (SUBJECT) of which there is no record (SUBJECT, binding, Prot00⟩ (the predicate and the object are the same as in the positive). They are "Prot00" and "Prot01 Note that "Prot03" in the fifth record is not included because the statement violates the condition. In the following, all the subjects may be used, and some of them may be selected randomly or by any other method. Suppose both "Prot00" and "Prot01" are selected. This step then stores a record ⟨SUBJECT, binding, Prot00⟩ for each subject into Negative Protein Context Statement Table 16. In the current example, the first two records in Table 16 are stored. Here the subject and the object in the first record are the same but such a record may be excluded for all the cases or for some specified predicates.*

*Step 803 also creates negative protein context statements for each positive protein context statement and stores them in Negative Protein Context Statement Table 16. Here the first record ⟨Kin00, binding, Prot00⟩ is used for explanation. This step first collects all the possible objects for each (OBJECT) of which there is no record ⟨Kin00, binding, OBJECT⟩ (the subject predicate and the predicate are the same as in the positive). They are "Kin00"; "Prot01", and "Prot02". In the following, all the objects may be used, and some of them may be selected randomly or by any other method. Suppose all of "Kin00", "Prot01" and "Prot02" are selected. This step then stores a record ⟨Kin00, binding, OBJECT⟩ for each object into Table 16. In the current example, the three records from the third to the fifth in Negative Protein Context Statement Table 16 are stored. Here the subject and the object in the third record are the same but such a record may be excluded for all the cases or for some specified predicates.*

*Step 804 discards from Negative Protein Context Statement Table 16 all the records appearing in Positive Protein Context Statement Table 13. In the current example, there is no such record and Table 16 is kept as it is.*

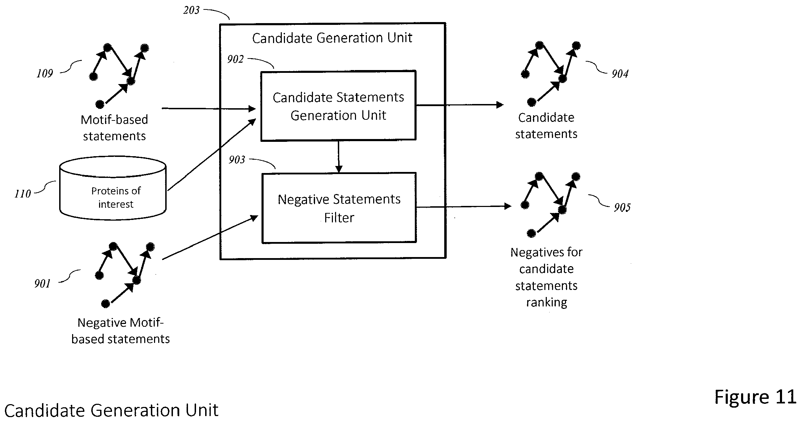

Figure 11 describes in details the Candidate Generator/Generation Unit 203 according to one embodiment.

This macro-component may be composed of the following specific components:

- **Candidate Statements Generation Unit 902** creates candidate statements to be considered during the prediction by the system. The candidate statements are based on the proteins kinases or substrates of interest 110 given as an input (or on all proteins in the positive statements if no specific list of interest is given). This unit inputs motif-based statements.
- **Negative Statements Filter (or negative candidate statements generation unit) 903** generates the negative candidate motif-based statements required by prediction processes. This is achieved by filtering the set of motif-based negative statements (901) so that for each candidate statement created by 902, only the negative motif-based statements that share the same predicate (motif) and either the subject or the object with it are present.

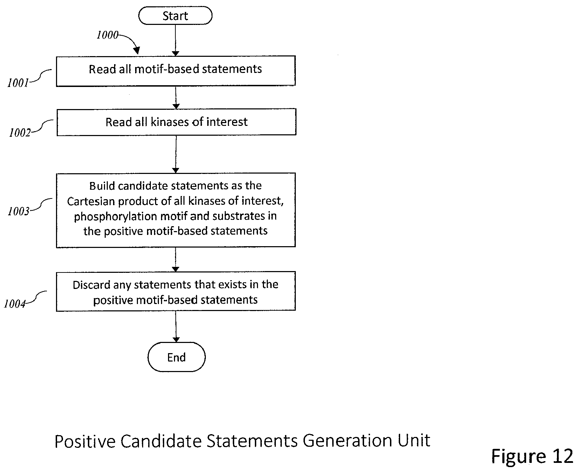

The flowchart in Figure 12 describes the process 902 of creation of candidate statements based on a list of proteins of interest:

- **1001.** This process starts by reading all positive motif-based statements 109.
- **1002.** Then the component reads all kinases of interest 110 (or all kinases).
- **1003.** The system creates candidate statements as the Cartesian product of all kinases of interest, phosphorylation motifs and protein substrates covered by the positive motif-based statements.
- **1004.** Finally, all candidate statements also present in the positive motif-based statements (i.e., including data for all known site-specific phosphorylation statements) are removed ensuring the prediction is not biased by statements used during the training phase.

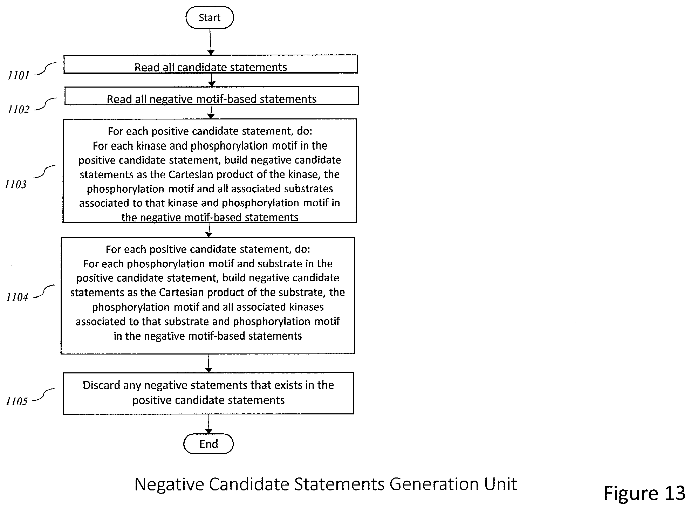

The flowchart in Figure 13 describes the process 903 of creation of negative candidate statements based on the list of positive ones:

- **1101.** This process starts by reading all candidate statements.

- **1102.** Then the component reads all negative motif-based statements 901.

- **1103.** For each protein kinase and phosphorylation motif in the candidate statements, the system creates negative candidate statements as the Cartesian product of the kinase, the phosphorylation motif and all associated substrates associated to that kinase and phosphorylation motif in the negative motif-based statements.

- **1104.** Similarly, for each phosphorylation motif and substrate in the candidate statements, the system creates negative candidate statements as the Cartesian product of the substrate, the phosphorylation motif and all associated kinases associated to that substrate and phosphorylation motif in the negative motif-based statements.

- **1105.** Finally, all negatives for candidate statements ranking also present in the candidate statements are removed.

**Example 9. Positive Candidate Statements Generation Unit**

*Step 1001 reads all the motif-based statements in Positive Motif-based Statement Table 17. Table 17 is a revised version of Table 12 for explaining this specific flowchart and has the 5 records as shown.*

**[Table 17] (revised version of Table 12)**

| Positive Motif-based Statement Table | | |
|---|---|---|
| **Subject (Kinase)** | **Predicate (Motif id)** | **Object (Substrate)** |
| Kin00 | TKL0 | Sub00 |
| Kin00 | TKL1 | Sub20 |
| Kin20 | TKL0 | Sub00 |
| Kin20 | AGC0 | Sub21 |
| Kin21 | TKL1 | Sub21 |

*Step 1002 reads all the kinases of interest in Kinase of Interest Table. An end user may specify them. Suppose Kinase of Interest Table 18, containing one kinase "Kin00", is given.*

**[Table 18]**

| Kinase of Interest Table |
|---|
| **Kinase** |

(continued)

| Kinase of Interest Table |
| --- |
| Kin00 |

For each kinase of interest, Step 1003 builds all possible combinations of a statement whose subject is the kinase, whose property is a motif id appearing in Positive Motif-based Statement Table 17, and whose object is a substrate appearing in Positive Motif-based Statement Table. Since there are three unique motif ids ("AGC0", "TKL0", and "TKL1') and three unique substrates ("Sub00', "Sub20', and "Sub21'), nine (=3 × 3) records are stored in Positive Candidate Table 19 as shown.

**[Table 19]**

| Positive Candidate Table | | | |
| --- | --- | --- | --- |
| Cand. id | Kinase | Motif id | Substrate |
|  | Kin00 | TKL0 | Sub00 |
|  | Kin00 | TKL0 | Sub20 |
|  | Kin00 | TKL0 | Sub21 |
|  | Kin00 | TKL1 | Sub00 |
|  | Kin00 | TKL1 | Sub20 |
|  | Kin00 | TKL1 | Sub21 |
|  | Kin00 | AGC0 | Sub00 |
|  | Kin00 | AGC0 | Sub20 |
|  | Kin00 | AGC0 | Sub21 |

Step 1004 discards from Positive Candidate Table 19 all the records appearing in Positive Motif-based Statement Table 17. In the current example, ⟨Kin00, TKL0, Sub00⟩ and ⟨Kin00, TKL1, Sub20⟩ appear in Table 17 and are discarded. As a result, Positive Candidate Table 19 becomes Positive Candidate Table 20 (each candidate id is also filled for later processing).

**[Table 20] (reprinted)**

| Positive Candidate Table | | | |
| --- | --- | --- | --- |
| Cand. Id | Subject [Kinase] | Predicate [Motif id] | Object [Substrate] |
| 100 | Kin00 | TKL0 | Sub20 |
| 101 | Kin00 | TKL0 | Sub21 |
| 102 | Kin00 | TKL1 | Sub00 |
| 103 | Kin00 | TKL1 | Sub21 |
| 104 | Kin00 | AGC0 | Sub00 |
| 105 | Kin00 | AGC0 | Sub20 |
| 106 | Kin00 | AGC0 | Sub21 |

## Example 10. Negative Candidate Statements Generation Unit

Step 1101 reads all the positive candidate statements in Positive Candidate Table 20.

Step 1102 reads all the negative motif-based statements in Negative Motif-based Statement Table 21.

**[Table 21] (revised version of Table 15) Negative Motif-based Statement Table**

| Subject [Kinase] | Predicate [motif id] | Object [Substrate] |
|---|---|---|
| Kin00 | TKL0 | Sub30 |
| Kin00 | TKL0 | Sub31 |
| Kin00 | TKL1 | Sub32 |
| Kin30 | TKL0 | Sub20 |
| Kin31 | TKL0 | Stub20 |
| Kin32 | AGC0 | Sub20 |

*Table 21 is a revised version of Table 15 for explaining this part of the method and has the 6 records as shown.*

*For each positive candidate, Step 1103 generates negative candidate statements and stores them in Negative Candidate Table 22.*

**[Table 22]**

| Negative Candidate Table | | | |
|---|---|---|---|
| Cand. Id | Subject [Kinase] | Predicate [Motif id] | Object [Substrate] |
| 100 | Kin00 | TKL0 | Sub30 |
| 100 | Kin00 | TKL0 | Sub31 |

*Suppose the statement with the candidate id "100" is to be processed. This step picks up, from Negative Motif-based Statement Table 21, statements whose subject and predicate are the same as the ones of the positive candidate, and stores them in Negative Candidate Table. In the current example, since the subject and the predicate of the "100" statement are "Kin00" and "TKL0", the first two records with "Kin00" and "TKL0" in Table 21 are picked up and stored in Table 22. Note that the third record is not picked up because the subjects are the same ("Kin00" but the predicates are different ("TKL0" and "TKL1").*

*For each positive candidate, Step 1104 generates negative candidate statements and stores them in Negative Candidate Table. Suppose the statement with the candidate id "100" is to be processed. This step picks up, from Negative Motif-based Statement Table 21, statements whose predicate and object are the same as the ones of the positive candidate, and stores them in Negative Candidate Table 22. In the current example, since the predicate and the object of the "100" statement are "TKL0" and "Sub20", the fourth and the fifth records with "TKL0" and "Subj20" in Table 21 are picked up and stored in Table 22. As a result, Table 22 becomes Table 23. Note that the sixth record is not picked up because the objects are the same ("Sub20") but the predicates are different ("TKL0" and "AGC0").*

**[Table 23]**

| Negative Candidate Table | | | |
|---|---|---|---|
| Cand. Id | Subject [Kinase] | Predicate [Motif id] | Object [substrate] |
| 100 | Kin00 | TKL0 | Sub30 |
| 100 | Kin00 | TKL0 | Sub31 |
| 100 | Kin30 | TKL0 | Sub20 |
| 100 | Kin31 | TKL0 | Sub20 |

*Step 1105 discards from Negative Candidate Table 23 all the records appearing in Positive Candidate Table 20. In the current example, there is no such record and Table 23 is kept as it is.*

**Relational Learning Unit**

The Relational Learning Unit 204 consists of an off-the-shelf statistical relational learning model (see Nickel et al) that learns from the positive and negative motif-based statements (outputs of the motif converter 105 and of the negative motif-based statements generator 202), and potentially from the positive and negative protein context statements (output of the protein context statements generator 107 and the negative protein context statements generator 201). The learned model is used for *link prediction,* i.e., to predict the existence of the candidate statements generated by candidate generator 203.

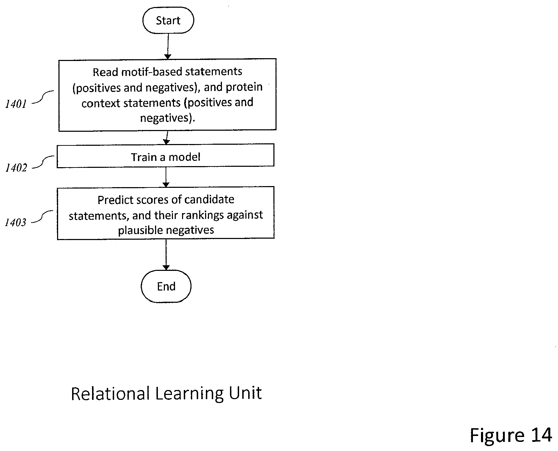

The flowchart in Figure 14 describes the process of the relational learning unit 204:

- **1401.** Read motif-based statements (positives 109 and negatives - output of negative statement generator 202), and protein context statements (positives 108 and negatives - output of negative statement generator 201).
- **1402.** Split motif-based statements in train and test sets. Add protein context statements to train set. Train the model. Training may include model selection over combinations of hyperparameters (and may adopt cross-validation), and may adopt a state-of-the-art, learning-to-rank evaluation protocol.

  In more detail, the selection is carried out by cross-validation over multiple embedding models and an exhaustive grid search over multiple combinations of hyperparameters. In each iteration of the cross-validation, the model is trained on a fraction of the phosphorylation network and tested on a disjoint validation set.

  Model selection chooses as best the model with the highest selected performance metric, which is typically a learning-to-rank metric such as the mean reciprocal rank (MRR). The adopted protocol is also based on learning-to-rank: each positive in the validation set is scored by the system, with a number of negatives that are either generated on the fly with an appropriate heuristic (e.g. by corrupting subjects or objects of positive relations), or retrieved from a gold standard. Positives and negatives scores are ranked, and ranks are used to compute the learning-to-rank performance metric (e.g. MRR).

- **1403.** The best performing model obtained at 1402 is used to predict the existence of positive candidate statements 904. Such existence is determined in a learning-to-rank fashion by the ranking of each of these statements against all plausible negatives 905 (such negatives share the same predicate and either the subject or the object). In addition to the rankings, the Relational Learning Unit may also output scores (such as normalised confidence weights), which may be used to implement custom ranking and filtering later on.

**Example 11. Relational Learning Unit**

*Step 1401 reads Positive Motif-based Statement Table, Negative Motif based Statement Table, Positive Protein Context Statement Table, and Negative Protein Context Statement Table. Examples of these tables are Table 12, Table 15, Table 13, Table 16 (all shown previously). These are just examples and the sizes of the tables are usually much bigger.*

*Step 1402 trains a model with a Relational Learning method. Any method satisfying the following conditions may be used.*

- *Includes a training phase based on known phosphorylation as converted to motif based statements and the negatives of these statements and a prediction phase using candidate statements*
- *In the training phase:*

  ○ *Inputs positive 3-ary (ternary) statements and negative 3-ary statements.*
  ○ *Trains a model, which put a score to a statement after fraining, by trying to put a higher score to each input positive statement and a lower score to each input negative statement as much as possible.*

- *In the prediction phase:*

  ○ *Inputs positive 3-ary candidate statements and negative candidate 3-ary statements that model facts known to be false in the real world. The negative candidate 3-ary statements may have been generated as corrupted versions of a positive statement (i.e. by replacing the subject or the object of the statement with something else that does not make sense biologically speaking),*
  ○ *Outputs, for each positive statement, a score and a score rank in the positive statement and the corresponding negative statements.*

*Examples of such methods are CP (*Frank L Hitchcock. "The expression of a tensor or a polyadic as a sum of products". In: Studies in Applied Mathematics 6.1-4 (1927), pp. 164-189*), DistMult (*Bishan Yang et al. "Embedding entities and relations for learning and inference in knowledge bases". In: arXiv preprint arXiv:1412.6575 (2014)*)), RESCAL (*Maximilian Nickel, Volker Tresp, and Hans-Peter Kriegel. "A three-way model for collective learning on multi-relational data". In: Proceedings of the 28th international conference on machine learning (ICML-11). 2011, pp. 809-816*), TransE (*Antoine Bordes et al. "Translating embeddings for modeling multi-relational data". In: Advances in neural information processing systems. 2013, pp. 2787-2795).*, ComplEx (*Theo Trouillon et al. "Complex embeddings for simple link prediction". In: arXiv preprint arXiv:1606.06357 (2016)*)), NTN (*Richard Socher et al. "Reasoning with neural tensor networks for knowledge base completion". In: Advances in neural information processing systems. 2013, pp. 926-934*), HoIE (*Maximilian Nickel, Lorenzo Rosasco, and Tomaso Poggio. "Holographic embeddings of knowledge graphs". In: arXiv preprint arXiv:1510.04935 (2015)) etc.*

*In the current example, suppose ComplEx is used and a model is learned with the method and the input data above (the 4 tables). The details of training are omitted.*

*Step 1403 predicts the rank and the score and stores them in Output Table for each positive candidate in Positive Candidate Table 20, shown previously.*

*The details of prediction are shown here. Suppose the "100" positive candidate in Table 20 is processed. Negative candidates corresponding to the positive candidate are the 4 records in Negative Candidate Table 23. Step 1403 first calculates a score for each of the 5 candidates. The score is computed by the inference (i.e. prediction) step of the model. During learning, the relational learning model learns the parameters of a scoring function that maps a 3-ary input to a real number (the score). The actual function depends on the adopted relational model (DistMult, and ComplEX use dot products, TransE uses vector norm, RESCAL uses matrix products, etc). Note that the score may or may not be a probability estimate. If it is not a probability estimate, it can be converted using a sigmoid function. Suppose the scores are 0.78 for the positive and 0.23, 0.85, 0.45, 0.87 for the 4 negatives. Then, the rank of the positive is 3 because 0.78 is the third largest score among the five scores. Step 1403 then stores 3 and 0.78 with the statement information in Output Table. The first record of Output Table 24 is the result of this process for "100". Similarly other candidates are processed and Output Table 24 is obtained.*

**[Table 24]**

| Output Table | | | | | |
|---|---|---|---|---|---|
| **Cand. Id** | **Kinase** | **Motif id** | **Substrate** | **Rank** | **Score** |
| 100 | Kin00 | TKL0 | Sub20 | 3 | 0.78 |
| 101 | Kin00 | TKL0 | Sutt21 | 10 | 0.55 |
| 102 | Kin00 | TKL1 | Sub00 | 2 | 0.65 |
| 103 | Kin00 | TKL1 | Sub21 | 8 | 0.17 |
| 104 | kin00 | AGC0 | Sub00 | 1 | 0.95 |
| 105 | Kin00 | AGC0 | Sub20 | 4 | 0.66 |
| 106 | Kin00 | AGC0 | Sub21 | 9 | 0.05 |

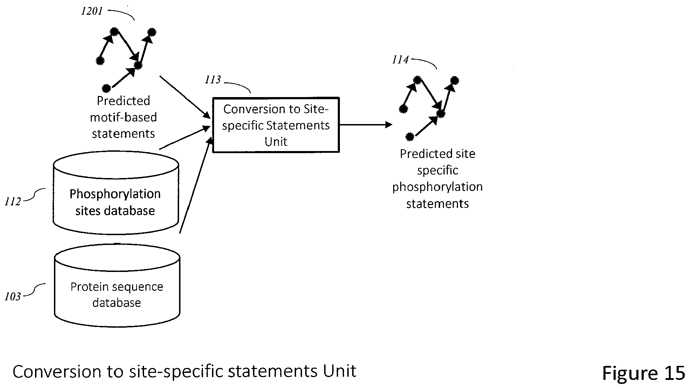

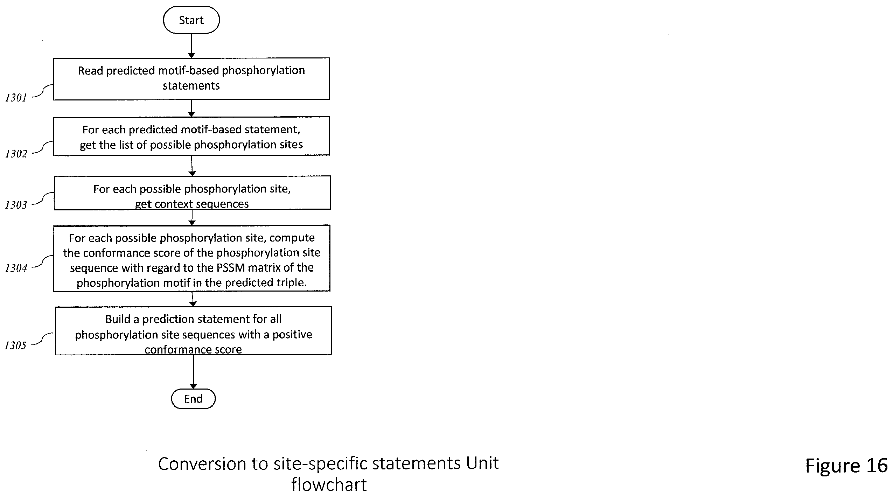

The flowchart in Figure 16 details the workings of the converter to site-specific statements 113, illustrated in Figure 15 which shows the relevant parts of Figure 1 and the input of the predicted motif-based statements, and describes the process of conversion of the predicted motif-based statements into predicted site-specific statements:

- **1301.** This process starts by reading all predicted motif-based phosphorylation statements 1201.
- **1302.** For each predicted motif-based statement, the system extracts a list of all possible phosphorylation sites for the substrate in the input statement from the phosphorylation sites database 112.
- **1303.** For each phosphorylation site, the system extracts the site context sequence from the protein sequence database 103.
- **1304.** For each possible phosphorylation site, the system computes the conformance score of the phosphorylation site sequence with regard to the phosphorylation motif in the predicted triple.
- **1305.** The system produces a set of transformed site-specific statements 114 where the kinase and substrate are as present in the predicted motif-based statement, and the sites are those that have a positive predicate motif conformance score. The score is attached to the statement scores produced by the Relational Learner/Learning Unit 204. These scores may be used for arbitrary ranking, filtering, etc., of the system results.

**Example 12.**

*Step 1301 reads predicted motif-based phosphorylation statements in Output Table 24.*

*Step 1302 gets the list of possible phosphorylation sites from Phosphorylation Sites Table 25 for each predicted motif-based phosphorylation statement and creates Motif Match Score Table 28. In the explanation below, only the first record (predicted motif-based phosphorylation statement) in Table 24 is used. Since the substrate is "Sub20" in the "100" record, the phosphorylation sites S269, S27 and T271 in the corresponding record in Phosphorylation Sites Table*

*25 are obtained.*

**[Table 25]**

| Phosphorylation Sites Table | |
|---|---|
| **Substrate** | **Phosphorylation sites** |
| Sub20 | {S85, S120, T97} |
| ... | ... |

*Then Step 1302 creates Motif Match Score Table 26.*

**[Table 26]**

| Motif Match Score Table | | | |
|---|---|---|---|
| **Cand. Id** | **Phosphorylation site** | **Context Sequence** | **Motif match score** |
| 100 | 585 | | |
| 100 | 5120 | | |
| 100 | T97 | | |
| ... | ... | | ... |

*Each record in Table 26 corresponds to Table 24 a phosphorylation site ("Cand. id" is also inserted for linking data between the Output Table and Motif Match Score Table). The empty fields "context sequence" and "motif match score" will be filled in later steps.*

*For each possible phosphorylation site, Step 1303 refers to Protein Sequence Table 27 and updates Motif Match Score Table 28.*

**[Table 27] Protein Sequence Table**

| (enriched version of Table 5) | |
|---|---|
| **Protein** | **Sequence** |
| Sub00 | MFQIPEFEPS EQEDSSSAER GLGPSPAGDG PSGSGKHHRQ APGLLWDASH QQEQFTSSSH HGGAGAVEIR SRHSSYPAGT EDDEGMGEEP SPFRGRSRSA PPNLWAAQRY GRELRRMSDE FVDSFKKGLP RPKSAGTATQ MRQSSSWTRV FQSWWDRNLG RGSSAPSQ |
| ... | ... |
| Sub20 | MYQAINPCPQ SWYGSPQLER EIVCKMSGAP HYPNYYPVHP NALGGAWFDT SLNARSLTTT PSLTTCTPPS LAACTPPTSL GMVDSPPHIN PPRRIGTLCF DFGSAKSPQR CECVASDRPS TTSNTAPDTY RLLITNSKTR KNNYGTCRLE PLTYGI |
| ... | ... |

*Table 27 is an enriched version of Table 5, with the "Sub20" record. Processing of the first record in Motif Match Score Table 26 is as follows:*

- *Since the substrate is "Sub20" (obtained from Output Table 24 by fhe candidate id "100"), the record for "Sub20" in Protein Sequence Table 27 is looked up. Similarly to Step 403, the corresponding sequence to the phosphorylation site "S85" is extracted. Concretely, the sequence from the 78th (=85 - 7) character to the 92nd (=85+7) character in the sequence "MYQAINPCPQ ...", that is "TSLGMVDSPPHINPP", is extracted and filled in Motif Match Score Table.*

*After processing all, Motif Match Score Table 26 becomes Motif Match Score Table 28.*

**[Table 28]**

| Motif Match Score Table | | | |
|---|---|---|---|
| Cand. Id | Phosphorylation site | Context sequence | Motif match score |
| 100 | 585 | TSLGMVLDSPPHINPP | |
| 100 | 5120 | CVASDRPSTTSNTAP | |
| 100 | T97 | NPPRROFTMVFDFGS | |
| ... | ... | | ... |

For each possible phosphorylation site, Step 1304 calculates the conformance score and updates Motif Match Score Table. Processing of the first record in Motif Match Score Table 28 is as follows:

- Since the motif id is "TKL0" (obtained from Output Table 24 by the candidate id "100") and the sequence is "TSLGMVDSPPHINPP" the conformance score of "TSLGMVDSPPHINPP" for the PSSM of fhe "TKL0" in Motif Table 8 is calculated similarly to Step 502. Suppose "32" is obtained. Then, Motif Match Score Table is updated as shown in the first record of Table 29.

After processing all, Motif Match Score Table 28 becomes Motif Match Score Table 29.

**[Table 29]**

| Motif Match Score Table | | | |
|---|---|---|---|
| Cand. Id | Phosphorylation site | Context sequence | Motif match score |
| 100 | 585 | TSLGIMVDSPPHINPP | 32 |
| 100 | 5120 | CVASDRPSTTSNTAP | -47 |
| 100 | T97 | NPPRRIGTLCFDFGS | 295 |
| ... | ... | | ... |

Step 1305 creates Prediction Table 30 based on Output Table 24 and Motif Match Score Table 29. For each record in Motif Match Score Table 29, a record is created in Table 30 as long as the motif match score is positive. This is the most relaxed strategy, generating statements with sites that have a higher chance than random for being associated with the predicate motif (sites with negative scores should never be included as they indicate disassociation). Another option (not shown here but perfectly possible with the embodiments) is to use just the site with the best positive score. This will put emphasis on high accuracy of the conversion, but may discard many potential valid predictions. An approach between these extremes is also feasible.

Suppose the first record in Table 29 is processed. Since the score is 32 and positive, the record is used. The kinase "Kin00", the substrate "Sub20", the rank "3", and the score "0.78" for "100" are taken from Table 24, and the phosphorylation site "S85" and the motif match score "32" are taken from Table 29, and the first record in Table 30 is created.

Next, suppose the second record in Table 29 is processed. Since the score is -47 and negative, the record is discarded.

Finally, Prediction Table 30 is used.

**[Table 30]**

| Prediction Table | | | | | |
|---|---|---|---|---|---|
| Kinase | Phosphorylation site | Substrate | Rank | Score | Motif match score |
| Kin00 | 585 | Sub20 | 3 | 0.78 | 32 |
| Kin00 | T97 | Sub20 | 3 | 0.78 | 296 |
| ... | ... | ... | ... | ... | ... |

Outputted Prediction Table 30 is supposed to be used by those who perform biological experiments. For

*example, with the first record in Table 30, a biologist is supposed to perform an experiment investigating whether "Kin00" really phosphorylates "S85" on "Sub20".*

*For such biologists, the rank, the score, and the motif match score may be used for deciding the priorities of experiments. For helping them, the system may sort and/or filter records in Prediction Table 30. For example, the threshold value "50" for the motif match score may be set for filtering out records with a motif match score lower than the value. Then, the first record is excluded because 32 < 50 and the second record is kept 296 > 50.*

**Scope**

By providing quantitative and ranked predictions of site-specific phosphorylation, the embodiments support the design of biomedical experiments aimed at discovery of functional impacts of phosphorylation pathways. The quality of the system's predictions is directly related to the quality of the input data. The system performs best with manually curated input, even though it may also cope with some level of noise in the data.

The system expects that all protein-related input databases (namely the Phosphorylation Network 102, the Protein database 103, the Kinase family database 104, the Kinases of interest 108 and the Protein context knowledge graph 106) use the same identification scheme to refer to the same entities (e.g. Protein kinase "Q02156" has the same identifier in all databases). This guarantees that a single entity, such as a protein, is represented as a single node in the knowledge graph used by the system. This is an important condition for the relational learning model to learn the features correctly from the knowledge graph, without extra conversion.

**Functionality, Effectiveness**

The embodiments provide an apparatus and a method to predict new phosphorylations from phosphorylation knowledge bases and potentially additional context information such as protein-protein interaction networks. The embodiments may yield reliable phosphorylation predictions in a more comprehensive manner than the prior art (i.e., for arbitrary combinations of known kinases, sites and substrates). Also, the approach is fully automated (even the optimal features are extracted autonomously) and thus may produce the results from publicly available data sets without any human intervention. This is another advantage when compared to prior art, which often requires extensive manual efforts in data preparation and feature engineering in order to work properly.

The embodiments may solve the three technical problems A), B) and C) as follows:

A. Embodiments may solve Technical Problem A by yielding phosphorylation predictions that leverage latent features extracted by a relational learning model that learns from the whole phosphorylation network (and, when available, additional context information).

B. Embodiments may solve Technical Problem B by overcoming the lack of models applicable to context-dependent data by converting phosphorylation knowledge bases into typed binary relations based on discovery of motifs specific to particular kinase families.

C. Embodiments may solve Technical Problem C by generating negatives based on the actual phosphorylation biology, which are much more reliable than negatives produced with the standard techniques.

Some embodiments have one or more of the following practical effects:

- **More efficient design of computational experiments.** Since all steps in the presented method are fully automated (including the feature engineering) and may be easily adjusted by tuning the module parameters, the technology may be used for many different set ups of phosphorylation prediction experiments. This may facilitate efficient identification of the models best complying with the requirements of the downstream laboratory experiments (e.g., maximising certain performance metrics or focusing on a limited sub-set of kinases).
- **Less time consuming and cheaper design of biomedical experiments aimed at discovery of functional impacts of phosphorylation pathways.** This is due to automated discovery of small numbers of (ranked) phosphorylation candidates, which may be used in much faster design of specific hypothetical phosphorylation networks to be verified by wet lab (physical laboratory) experiments.
- **More flexibility in designing the wet lab experiments.** As the embodiments may easily generate prediction candidates for any type of phosphorylation, they may be used for a broad range of experimental designs covering all known kinases and/or substrates off-the-shelf, which is not so straightforward with the prior art.
- **More comprehensive support of contextual information.** The embodiments may easily incorporate additional knowledge graphs with relevant information on the kinases or substrates (e.g., protein interaction networks or protein databases). This allows biomedical researchers to utilise additional sources of facts relevant to phosphorylation with an unprecedented ease, leading to more focused and reliable prediction results.

Embodiments may provide a system that may automatically:

- combine phosphorylation data with related protein contextual data, in the form of knowledge graphs (see discovery system 101), such as the protein context knowledge graph;

- predict previously unknown site-specific phosphorylation statements from the combined data using a link prediction technique (see link prediction unit 111).

These features address Technical Problem A.

Embodiments may provide a system that may realise two-way conversion between site-specific phosphorylation statements and actionable triples with generalised predicates based on motifs (converters 105, 113). These features address Technical Problem B.

Existing link prediction techniques cannot produce meaningful results from the phosphorylation networks. The inventors have come to the realisation that the data may be converted to an internal format in order to make predictions and then converted back to the standard site-specific phosphorylation statements so that users may benefit from the predictions.

Finally, embodiments may provide a system that may realise generation of negative motif-based phosphorylation statements that may be safely used for link prediction (using Negative Motif-based Statements Generation Unit 202). These features address Technical Problem C. Step 702 and Step 703 in Figure 9 are inventive both taken separately or together in the way they create negative motif-based statements by substrate and/or by kinase.

Applicable link prediction techniques require reliable negative examples that cannot be safely generated with the techniques typically used in prior art.

**Computer Implementation**

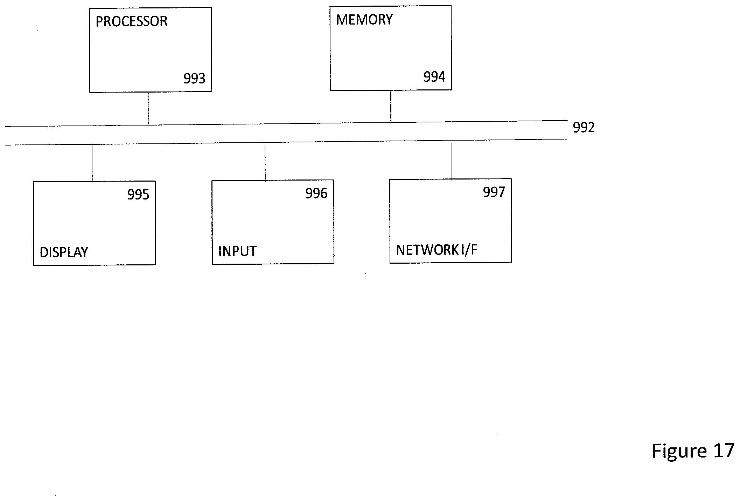

Figure 17 is a block diagram of a computing device, such as a data storage server which embodies the present invention, and which may be used to implement a method of finding specific phosphorylation sites. The computing device comprises a processor 993, and memory, 994. Optionally, the computing device also includes a network interface 997 for communication with other computing devices, for example with other computing devices of invention embodiments, or for connection to the phosphorylation data set/network 102, protein sequence database 103 and kinase family database 104 used in embodiments. Alternatively, this information may be stored in local data storage.

For example, an embodiment may be composed of a network of such computing devices. Optionally, the computing device also includes one or more input mechanisms such as keyboard and mouse 996, for example to input proteins of interest 110, and a display unit such as one or more monitors 995, which may display results such as Table 30 to the user. The components are connectable to one another via a bus 992.

The memory 994 may include a computer readable medium, which term may refer to a single medium or multiple media (e.g., a centralized or distributed database and/or associated caches and servers) configured to carry computer-executable instructions or have data structures stored thereon. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding or carrying a set of instructions for execution by the machine and that cause the machine to perform any one or more of the methods of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing code stored in the memory to implement the various different functions of the modules (the converters 105, 103, the link predictor 111 their subcomponents) described here and in the claims. The memory 994 stores data being read and written by the processor 993. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and steps discussed herein.

The display unit 997 may display a representation of data stored by the computing device (for example the various statements input and created) and may also display a cursor and dialog boxes and screens enabling interaction between a user and the programs and data stored on the computing device. The input mechanisms 996 may enable a user to input data and instructions to the computing device, such as any parameters for the relational learner and any proteins of interest.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball etc may be included in the computing device.

The converters 105, 113 may comprise processing instructions stored on a portion of the memory 994, the processor 993 to execute the processing instructions, and a portion of the memory 994 to store the statements during the execution of the processing instructions for conversion between motif-based and site-specific statements. The converted statements may be stored on the memory 994 and/or on a connected storage unit.

The link predictor 111 may comprise processing instructions stored on a portion of the memory 994, the processor 993 to execute the processing instructions, and a portion of the memory 994 to store the statements during the execution of the processing instructions for producing candidate statements, negative statements, negative candidate statements and predicted statements. The converted statements may be stored on the memory 994 and/or on a connected storage unit.

Methods embodying the present invention may be carried out on a computing device such as that illustrated in Figure 17. Such a computing device need not have every component illustrated in Figure 17, and may be composed of a subset of those components. A method embodying the present invention may be carried out by a single computing device in communication with one or more data storage servers via a network. The computing device may be a data storage itself storing the various statements including the final predicted statements and their scores.

A method embodying the present invention may be carried out by a plurality of computing devices operating in cooperation with one another. One or more of the plurality of computing devices may be a data storage server storing at least a portion of various statements.

### *Glossary*

Kinase

"A type of enzyme (a protein that speeds up chemical reactions in the body) that adds chemicals called phosphates to other molecules, such as sugars or proteins. This may cause other molecules in the cell to become either active or inactive. Kinases are a part of many cell processes. Some cancer treatments target certain kinases that are linked to cancer." https://www.cancer.gov/publications/dictionaries/cancer-terms?CdrID=641114

Phosphorylation

Phosphorylation (the addition of a phosphoryl group $PO_3$) is one of the most important protein interactions essential to many major signalling pathways in all live organisms. It is a directed and context-dependent relation - an agent (*kinase*) protein K phosphorylates a patient (*substrate*) protein S at a site *L* (a *location* within the amino acid chain of the substrate protein). Phosphorylation regulates the behaviour of the substrate protein - it may be activated or deactivated, or its function may be modified in a more complex way. The downstream impact of phosphorylation pathways often results in major changes in the behaviour of the corresponding cells - the cells may start growing, proliferating or enter the process of programmed cell death (apoptosis).

Phosphorylation Site

A location (at an amino acid) within the amino acid chain of the substrate protein.

Phosphorylation Network

A knowledge base of (human or other animal) phosphorylation reactions, made of relations between kinases and specific phosphorylation sites in substrates.

Substrate

"In chemistry, a substrate is typically the chemical species being observed in a chemical reaction, which reacts with reagent to generate a product". https://en.wikipedia.org/wiki/Substrate (chemistry)

Signalling pathway

"Describes a group of molecules in a cell that work together to control one or more cell functions, such as cell division or cell death. After the first molecule in a pathway receives a signal, it activates another molecule. This process is repeated until the last molecule is activated and the cell function is carried out. Abnormal activation of signaling pathways may lead to cancer, and drugs are being developed to block these pathways. These drugs may help block cancer cell growth and kill cancer cells." https://www.cancer.gov/publications/dictionaries/cancer-terms?cdrid=561720

Motif

A common (or consensus) sequence pattern of a protein, with a likely biological function. It may be a part or all of a protein sequence and in this context is derived as a consensus from a group of sequences. It may be represented as a "position-specific scoring matrix" or PSSM. A motif may provide the log likelihood score for each amino acid at a given position in a sequence.

Site-specific statement

A site-specific statement represents a phosphorylation interaction between a protein kinase and a substrate kinase along with the specific amino acid residue (site location) where the phosphorylation happen on the protein residue. It is assumed that the statement is of the triple form set out below, but it may form a longer tuple.
⟨ *protein kinase, phosphorylation site location, protein substrate*⟩.

Motif-based statement

A motif-based statement represents a phosphorylation interaction between a protein kinase and a protein substrate with an abstract (non-example specific) notion of phosphorylation site: motif. This motif is learned from regularities in phosphorylation site sequence among a kinase family. A motif provides the log likelihood score for each amino acid at a given position in a sequence. Motif-based statements are of the tuple form as above: ⟨*protein kinase, motif, protein substrate*⟩.

Protein context statement

A protein context statement is a relational statement related to protein kinases and protein substrates such as additional protein-protein interactions. It comes in the form of a tuple, as for the other statements:

⟨*subject, relation, object*⟩,

indicating that *subject* and *object* entities hold a relationship *relation.*

Candidate statement

Candidate statements are the statements to be considered during the prediction step by the system. Candidate statements may be based on the kinases of interest given as an input by the user. They have the same form as a motif-based statement. They may include information as to a probability of the candidate statement.

Knowledge Graph

A collection of items (represented by *nodes*) any of which may be connected by links (i.e. binary relations) between them. They may be understood as a graph-structured knowledge base/database.

Statistical relational learning

A machine learning branch in which a feature vector representing an input item may contain its relationships to other input items.

Link Prediction

A technique that attempts to estimate the likelihood of the existence of a link between two nodes, based on observed links and attributes of nodes.

**Claims**

1. A system for discovery of predicted site-specific protein phosphorylation statements, the statements linking a kinase, a predicted phosphorylation site, and a protein substrate, the system comprising:

   a motif converter which is to receive inputs from:

   a phosphorylation data set storing known site-specific statements each in a site-specific format linking a kinase, a phosphorylation site and a protein substrate;
   a kinase family database; and
   a protein sequence database,

   the motif converter being arranged to produce motif-based phosphorylation statements each in a generalised format linking a kinase, a motif in the form of a generalised scoring matrix for amino acids at a phosphorylation site and to either side of the phosphorylation site; and a protein substrate;
   a link predictor to predict new motif-based statements, the link predictor including:

   a negative motif-based statements generator to generate negative statements from the motif-based statements;
   a candidate generator to generate candidate statements in the generalised format by combination of the motif-based statements and to generate negative candidate statements by combination of the candidate statements with the negative statements; and
   a relational learner to use the candidate statements and the negative candidate statements in a statistical relational learning model to score the candidate statements and retain predicted motif-based phosphorylation statements in the generalised format; and

   a site-specific converter to receive inputs from the protein sequence database and to receive the predicted motif-based phosphorylation statements in the generalised format and which is arranged to produce predicted site-specific phosphorylation statements in the site-specific format.

2. A system according to claim 1, wherein
   the motif converter is to:

   associate each site-specific statement with a kinase family in the kinase family database;
   use the protein sequence database to form a context sequence of an amino acid at the phosphorylation site for each site-specific statement and a plurality of amino acids to either side of the phosphorylation site;
   group the context sequences by kinase family;
   for the context sequences in each kinase family, compute one or more position-specific scoring matrices each of which provides a score for each amino acid at each position of the context sequence length, each position-specific scoring matrix defining a motif;
   for each site-specific statement, compute the conformance between the context sequence for that site-specific statement and each motif in the kinase family in which the context sequence is grouped; and to

   for each site-specific statement, create a motif-based statement linking the kinase, the best performing motif and the protein substrate.

3. A system according to any of the preceding claims, wherein the negative motif-based statements generator is to create negative statements by one or more of: replacing the substrate in a motif-based statement <K, M, S> with a different substrate and replacing the kinase in a motif-based statement with a kinase from a different kinase family to create a negative statement.

4. A system according to claim 3, wherein the substrate S in a motif-based statement <K, M, S> is replaced by a different substrate S' in that for each phosphorylation site on the different substrate, the context sequence is compared with the motif of the motif-based statement, and if the conformance is negative, a negative statement <K, M, S'> is provided.

5. A system according to claim 3 or 4, wherein the kinase K in a motif-based statement <K, M, S> is replaced by a different kinase K' in that for each kinase which is not in the same family as kinase K, a statement <K', M, S> is provided.

6. A system according to any of the preceding claims, wherein the negative motif-based statements generator is configured to compare the negative statements and the motif-based statements and to delete from the negative statements any statement that appears both as one of the negative statements and as one of the motif-based statements.

7. A system according to any of the preceding claims, wherein a negative statements filter in the candidate generator is also to, for each kinase and motif in a candidate statement, create negative candidate statements as the combination of the kinase, the motif and all substrates associated with the kinase and the motif in the negative statements.

8. A system according to any of the preceding claims, wherein a negative statements filter in the candidate generator is also to, for each motif and substrate in a candidate statement, create negative candidate statements as the combination of the motif, the substrate and all kinases associated with the substrate and the motif in the negative statements.

9. A system according to claim 6, 7 or 8, wherein the negative statements filter is to discard from the negative candidate statements any statements that appears both as one of the negative candidate statements and as one of the candidate statements.

10. A system according to any of the preceding claims, wherein the relational learner is to input motif-based statements and negative statements and to learn a model from these inputs which is then used to score the candidate statements and the negative candidate statements.

11. A system according to any of the preceding claims, wherein the candidate generator is to create candidate statements as the combination of every kinase included in the motif-based statements with every motif included in the motif-based statements and every substrate included in the motif-based statements minus the motif-based statements themselves.

12. A system according to any of the preceding claims, wherein the relational learner is to score and rank a candidate statement and all the negative candidate statements with the same kinase and motif or with the same motif and substrate and to output the rank and score of the candidate statement.

13. A system according to any of the preceding claims, further comprising a protein statements generator to receive input from a protein context database linking proteins by their interactions and to produce protein context statements, which are for use in the link predictor.

14. A system according to any of the preceding claims, wherein the site-specific converter is to:

input the predicted motif-based phosphorylation statements;
for each predicted motif-based phosphorylation statements extract a list of possible phosphorylation sites from a phosphorylation sites database;
for each phosphorylation site, extract the site context sequence from the protein sequence database and compute the conformance of the site context sequence with the motif in the predicted motif-based phosphorylation statement;
where the conformance for the phosphorylation site is above a predefined score, produce a predicted site-specific phosphorylation statement in the site-specific format linking the kinase and the substrate in the predicted motif-based statement with the phosphorylation site.

15. A method for discovery of predicted site-specific protein phosphorylation statements, the statements linking a kinase, a predicted phosphorylation site, and a protein substrate, the method comprising:

using data from:

> a phosphorylation data set storing known site-specific statements each in a site-specific format linking a kinase, a phosphorylation site and a protein substrate;
> a kinase family database; and
> a protein sequence database,

to produce motif-based phosphorylation statements each in a generalised format linking a kinase, a motif in the form of a generalised scoring matrix for amino acids at a phosphorylation site and to either side of the phosphorylation site; and a protein substrate;
generating negative statements from the motif-based statements;
generating candidate statements in the generalised format by combining the motif-based statements;
generating negative candidate statements by combination of the candidate statements with the negative statements;
using the candidate statements and negative candidate statements in a statistical relational learning model to score the candidate statements and retain predicted motif-based phosphorylation statements in the generalised format;
receiving data inputs from the protein sequence database and the predicted motif-based phosphorylation statements in the generalised format and producing predicted site-specific phosphorylation statements in the site-specific format.

16. A method according to claim 15, further comprising testing whether there is phosphorylation as predicted by the predicted site-specific phosphorylation statements in a physical laboratory to verify whether the prediction is correct.

17. A computer program which when executed on a computer apparatus carries out the method of claim 15.

System Overview Block Diagram —Tech. Prob. A & B

Figure 1A

Phosphorylation
Data set <K, site, S.

Kinase data ————▶

Protein sequence data ————▶

| Produce motif-based statements <K, M, S> | S10 |

Generate candidate statements by combining K, M & S from different statements — S20

Generate negative candidate statements by negating and combining motif-based statements — S30

Scoring candidate statements to retain predicted motif-based statements — S40

Protein sequence data ————▶ Produce predicted site-specific statements <K, site, S> — S50

Figure 1B

System Overview Block Diagram –Tech. Prob. A & B

Figure 1C

System Overview Block Diagram –Tech. Prob. C

Figure 2

Figure 3

Conversion to motif-based statements Unit

Conversion to motif-based statements Unit

Motif-based
Statements
generation Unit

Motif Discovery Unit

Phosphorylation
network

Protein
sequence
database

Kinase family
database

Motif-based
statements

```
                    ┌─────────┐
                    │  Start  │
                    └─────────┘
                         │
                         ▼
        ┌────────────────────────────────────┐
        │   Read site-specific statements from│
  401   │      phosphorylation network        │
        └────────────────────────────────────┘
                         │
                         ▼
        ┌────────────────────────────────────┐
        │ For each site-specific statement, add family │
  402   │  of the kinase from kinase family database    │
        └────────────────────────────────────┘
                         │
                         ▼
        ┌────────────────────────────────────┐
        │ For each site-specific statement, add site    │
        │ context amino acid sequence information        │
  403   │    from protein sequence database              │
        └────────────────────────────────────┘
                         │
                         ▼
        ┌────────────────────────────────────┐
        │  Group phosphorylation sequences per │
  404   │           kinase family              │
        └────────────────────────────────────┘
                         │
                         ▼
        ┌────────────────────────────────────┐
        │ For each kinase family, compute position- │
  405   │  specific scoring matrices (= motifs).     │
        └────────────────────────────────────┘
                         │
                         ▼
                    ┌─────────┐
                    │   End   │
                    └─────────┘
```

Motif Discovery Unit flowchart

Figure 4

Start

Read site-specific statements with kinase family and site context sequence information

501

For each statement, compute the conformance with every motif obtained from the same kinase family as the kinase in the statement.

502

Discard motifs candidates that has negative conformance with a given statement

503

For each statement, generate a motif-based statement of the form <Kinase, Motif_ID, Substrate> for the best conformant motif among the motifs from the same family as the kinase in the site-specific statement

504

End

Motif-based Statement Generation Unit flowchart

Figure 5

Knowledge Base Enrichment Unit

Figure 6

Start

Read motif-based statements

601

Get neighbour statements of kinase and
substrate within a given distance from
protein context knowledge graph

602

End

Kin00 — binding → Prot00

Prot00 — reaction → Prot02

Prot00 — ptmod

binding

Prot01 — catalysis

Prot03

Protein Context Statements Generation Unit

Figure 7

Link Prediction Unit

Figure 8

```
                    ┌─────────┐
                    │  Start  │
                    └─────────┘
                         │
                         ▼
        ┌──────────────────────────────────┐
   701  │   Read all motif-based statements │
        └──────────────────────────────────┘
                         │
                         ▼
        ┌──────────────────────────────────┐
        │  For each input statement <K, M, S>, │
   702  │  create a set of corresponding negative │
        │  motif-based statements <K, M, S'>  │
        └──────────────────────────────────┘
                         │
                         ▼
        ┌──────────────────────────────────┐
        │  For each input statement <K, M, S>, │
   703  │  create a set of corresponding negative │
        │  motif-based statements <K', M, S>  │
        └──────────────────────────────────┘
                         │
                         ▼
        ┌──────────────────────────────────┐
        │  Discard any negative statement that │
   704  │  exists in the input positive statements │
        └──────────────────────────────────┘
                         │
                         ▼
                    ┌─────────┐
                    │   End   │
                    └─────────┘
```

Negative Motif-based Statements
Generation Unit flowchart

Figure 9

```
                                    ┌─────────┐
                                    │  Start  │
                                    └─────────┘
                                         │
                                         ▼
              ┌──────────────────────────────────────────────────┐
     801      │           Read all protein context statements     │
              └──────────────────────────────────────────────────┘
                                         │
                                         ▼
              ┌──────────────────────────────────────────────────┐
              │              For each input statement,            │
              │   build a new negative statement replacing only   │
     802      │      the subject element by a randomly picked     │
              │        subject from the positive statements:      │
              │          <new_subject, relation, object>          │
              └──────────────────────────────────────────────────┘
                                         │
                                         ▼
              ┌──────────────────────────────────────────────────┐
              │              For each input statement,            │
              │   build a new negative statement replacing only   │
     803      │   the object element by a randomly picked object  │
              │  from the positive statements: <subject, relation,│
              │                   new_object>                     │
              └──────────────────────────────────────────────────┘
                                         │
                                         ▼
              ┌──────────────────────────────────────────────────┐
     804      │   Discard any negative statement that exists      │
              │        in the input positive statements           │
              └──────────────────────────────────────────────────┘
                                         │
                                         ▼
                                    ┌─────────┐
                                    │   End   │
                                    └─────────┘
```

Negative Protein Context Statements
Generation Unit

Figure 10

Figure 11

Candidate Generation Unit

Start

*1000*

Read all motif-based statements
*1001*

Read all kinases of interest
*1002*

Build candidate statements as the
Cartesian product of all kinases of interest,
phosphorylation motif and substrates in
the positive motif-based statements
*1003*

Discard any statements that exists in the
positive motif-based statements
*1004*

End

Positive Candidate Statements Generation Unit

Figure 12

Start

1101 — Read all candidate statements

1102 — Read all negative motif-based statements

1103 — For each positive candidate statement, do:
For each kinase and phosphorylation motif in the positive candidate statement, build negative candidate statements as the Cartesian product of the kinase, the phosphorylation motif and all associated substrates associated to that kinase and phosphorylation motif in the negative motif-based statements

1104 — For each positive candidate statement, do:
For each phosphorylation motif and substrate in the positive candidate statement, build negative candidate statements as the Cartesian product of the substrate, the phosphorylation motif and all associated kinases associated to that substrate and phosphorylation motif in the negative motif-based statements

1105 — Discard any negative statements that exists in the positive candidate statements

End

Negative Candidate Statements Generation Unit

Figure 13

```
                          ┌─────────┐
                          │  Start  │
                          └─────────┘
                               │
                               ▼
            ┌──────────────────────────────────────┐
            │       Read motif-based statements      │
            │   (positives and negatives), and protein│
    1401 ──│    context statements (positives and    │
            │              negatives).                │
            └──────────────────────────────────────┘
                               │
                               ▼
            ┌──────────────────────────────────────┐
    1402 ──│            Train a model               │
            └──────────────────────────────────────┘
                               │
                               ▼
            ┌──────────────────────────────────────┐
            │       Predict scores of candidate      │
    1403 ──│  statements, and their rankings against │
            │           plausible negatives          │
            └──────────────────────────────────────┘
                               │
                               ▼
                          ┌─────────┐
                          │   End   │
                          └─────────┘
```

Relational Learning Unit

Figure 14

Conversion to site-specific statements Unit

Figure 15

Start

Read predicted motif-based phosphorylation
statements

*1301*

For each predicted motif-based statement,
get the list of possible phosphorylation sites

*1302*

For each possible phosphorylation site,
get context sequences

*1303*

For each possible phosphorylation site, compute
the conformance score of the phosphorylation site
sequence with regard to the PSSM matrix of the
phosphorylation motif in the predicted triple.

*1304*

Build a prediction statement for all
phosphorylation site sequences with a positive
conformance score

*1305*

End

Conversion to site-specific statements Unit
flowchart

Figure 16

| PROCESSOR | | MEMORY | |
|---|---|---|---|
| | 993 | | 994 |

992

| | 995 | | 996 | | 997 |
|---|---|---|---|---|---|
| DISPLAY | | INPUT | | NETWORK I/F | |

Figure 17

**EP 3 404 567 A1**

Europäisches
Patentamt

European
Patent Office

Office européen
des brevets

# EUROPEAN SEARCH REPORT

Application Number

EP 17 17 1925

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | NEUBERGER GEORG ET AL: "pkaPS: prediction of protein kinase A phosphorylation sites with the simplified kinase-substrate binding model", BIOLOGY DIRECT, BIOMED CENTRAL LTD, LO, vol. 2, no. 1, 12 January 2007 (2007-01-12), page 1, XP021025705, ISSN: 1745-6150, DOI: 10.1186/1745-6150-2-1 * D1 page 2 of 23 paragraph 4 D1 page 7 of 23 paragraph 2 D1 page 13 of 23, paragraph 1; table 4 * | 1-17 | INV. G06F19/10 G06F19/24 G06F19/28 |
| Y | PIETER KELCHTERMANS ET AL: "Machine learning applications in proteomics research: How the past can boost the future", PROTEOMICS, vol. 14, no. 4-5, 1 March 2014 (2014-03-01), pages 353-366, XP055405990, DE ISSN: 1615-9853, DOI: 10.1002/pmic.201300289 * the whole document * | 1-17 | TECHNICAL FIELDS SEARCHED (IPC) G06F |

-/--

The present search report has been drawn up for all claims

| Place of search | Date of completion of the search | Examiner |
|---|---|---|
| Munich | 15 September 2017 | Rivera, Pedro V. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

56

Europäisches
Patentamt

European
Patent Office

Office européen
des brevets

# EUROPEAN SEARCH REPORT

Application Number

EP 17 17 1925

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | O. BUZKO ET AL: "A kinase sequence database: sequence alignments and family assignment", BIOINFORMATICS., vol. 18, no. 9, 1 September 2002 (2002-09-01), pages 1274-1275, XP055405747, GB ISSN: 1367-4803, DOI: 10.1093/bioinformatics/18.9.1274 * the whole document * | 1-17 | |
| A | AMOS BAIROCH ET AL: "The SWISS-PROT protein sequence data bank", NUCLEIC ACIDS RESEARCH, vol. 19, no. Supplement, 25 April 1991 (1991-04-25), pages 2247-2249, XP055405809, DOI: https://doi.org/10.1093/nar/19.suppl.2247 * the whole document * | 1-17 | |
| A | EP 1 339 006 A1 (AGELAB PHARMA GMBH [DE]) 27 August 2003 (2003-08-27) * the whole document * | 1-17 | TECHNICAL FIELDS SEARCHED (IPC) |

The present search report has been drawn up for all claims

| Place of search | Date of completion of the search | Examiner |
|---|---|---|
| Munich | 15 September 2017 | Rivera, Pedro V. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 17 1925

This annex lists the patent family members relating to the patent documents cited in the above-mentioned European search report.
The members are as contained in the European Patent Office EDP file on
The European Patent Office is in no way liable for these particulars which are merely given for the purpose of information.

15-09-2017

| Patent document cited in search report | | Publication date | Patent family member(s) | | Publication date |
|---|---|---|---|---|---|
| EP 1339006 | A1 | 27-08-2003 | EP<br>US | 1339006 A1<br>2004023300 A1 | 27-08-2003<br>05-02-2004 |

EPO FORM P0459

For more details about this annex : see Official Journal of the European Patent Office, No. 12/82

# EP 3 404 567 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MAXIMILIAN NICKEL et al.** A review of relational machine learning for knowledge graphs. *arXiv preprint arXiv: 1503.00759,* 2015
- **MATT GARDNER ; TOM M MITCHELL.** Efficient and Expressive Knowledge Base Completion Using Subgraph Feature Extraction. *EMNLP,* 2015, 1488-149
- Knowledge Base Completion via Search-based Question Answering. **ROBERT WEST et al.** Proceedings of the 23rd International Conference on World Wide Web. WWW '14. ACM, 2014, 515-526
- **FRANK L HITCHCOCK.** The expression of a tensor or a polyadic as a sum of products. *Studies in Applied Mathematics,* 1927, vol. 6.1-4, 164-189
- **BISHAN YANG et al.** Embedding entities and relations for learning and inference in knowledge bases. *arXiv preprint arXiv:1412.6575,* 2014

- **MAXIMILIAN NICKEL ; VOLKER TRESP ; HANS-PETER KRIEGEL.** A three-way model for collective learning on multi-relational data. *Proceedings of the 28th international conference on machine learning (ICML-11),* 2011, 809-816
- **ANTOINE BORDES et al.** Translating embeddings for modeling multi-relational data. *Advances in neural information processing systems,* 2013, 2787-2795
- **THEO TROUILLON et al.** Complex embeddings for simple link prediction. *arXiv preprint arXiv:1606.06357,* 2016
- **RICHARD SOCHER et al.** Reasoning with neural tensor networks for knowledge base completion. *Advances in neural information processing systems,* 2013, 926-934
- **MAXIMILIAN NICKEL ; LORENZO ROSASCO ; TOMASO POGGIO.** Holographic embeddings of knowledge graphs. *arXiv preprint arXiv:1510.04935,* 2015